# EUROPEAN PATENT APPLICATION

(11) **EP 4 321 535 A1**
(43) Date of publication of application: **14.02.2024**
(21) Application number: 22784923.9
(22) Date of filing: 05.04.2022
(51) Int. Cl.: C07K 16/28, A61K 47/68, A61P 35/00, A61K 39/00

(54) **ANTI-CNTN4 ANTIBODY AND USE THEREOF**

(30) Priority: 06.04.2021 KR 20210044840
(71) Applicant: Genome and Company, Seongnam-si, Gyeonggi-do 13486 (KR)
(72) Inventor: JEON, Bu Nam, Seongnam-si, Gyeonggi-do 13486 (KR); CHUNG, Joo Yeon, Seongnam-si, Gyeonggi-do 13486 (KR); MOON, Soo Jung, Seongnam-si, Gyeonggi-do 13486 (KR); KIM, Hyunuk, Seongnam-si, Gyeonggi-do 13486 (KR); CHA, Mi Young, Seongnam-si, Gyeonggi-do 13486 (KR); CHUNG, Junho, Seongnam-si, Gyeonggi-do 13590 (KR); JIN, Junyeong, Gwacheon-si, Gyeonggi-do 13835 (KR); KIM, Soohyun, Seoul 03127 (KR); CHAE, Jisu, Seoul 02829 (KR)
(74) Representative: Dehns
(86) International application number: PCT/KR2022/004890
(87) International publication number: WO 2022/216014

(57) **Abstract**

The present invention relates to an anti-CNTN4 antibody or an antigen-binding fragment thereof, a pharmaceutical composition containing the same, and a use thereof for activating T cells upregulating cell-mediated immune responses, for example, for treating cancer.

## Description

### Technical Field

The present invention relates to an anti-CNTN4 antibody or an antigen-binding fragment thereof, a pharmaceutical composition containing the same, and a use thereof for activating T cells, for example, for treating cancer.

### Background Art

The human body has a defense system that protects itself from external invaders (viruses, toxins, or the like) and internal harmful changes (cancer cell mutations). Unlike normal cells, cancer cells have specific antigens on the surface thereof and are destroyed by the immune system at an early stage of cancer development. Then, when the balance of the power of cancer cells, which want to proliferate indefinitely, and immune cells, which want to attack the cancer cells, collapses, the cancer cells begin to proliferate substantially. When cancer cells grow further, they disturb the immune system in the body, and some cancer cells avoid immunity by using the immune checkpoint of immune cells, and when immune checkpoint inhibitors suppress the immune checkpoint, the power of immune cells increases, thereby killing the cancer cells.

Immune checkpoint inhibitors are drugs that attack cancer cells by activating T cells by blocking the activation of immune checkpoint proteins involved in T cell inhibition, and include CTLA-4, PD-1, PD-L1 inhibitors, or the like. Representative drugs, which are currently commercially available, include ipilimumab (product name: YERVOY^{®}) as CTLA-4 monoclonal antibodies, nivolumab (product name: OPDIVO^{®}) and pembrolizumab (product name: KEYTRUDA^{®}) as PD-1 monoclonal antibodies, and atezolizumab (product name: TECENTRIQ^{®}) and durvalumab (product name: IMFINZI^{®}) as PD-L1 monoclonal antibodies.

However, there are still carcinomas that are not treated with existing immune checkpoint inhibitors, and thus the development of new anticancer treatments is required.

### Disclosure of the Invention

### Technical Problem

The purpose of the present invention is to provide an anti-CNTN4 antibody or an antigen-binding fragment thereof, which specifically binds to a CNTN4 protein. In particular, the present invention is intended to provide an antibody or an antigen-binding fragment thereof that binds to the CNTN4 protein to neutralize an immune escape mechanism of CNTN4.

The present invention is also intended to provide a composition for preventing or treating diseases, such as cancer, caused by a decrease in T cell activity, by using the antibody or antigen-binding fragment thereof to block the immune escape mechanism of CNTN4 and activate T cells.

The present invention is also intended to provide a composition for analyzing or detecting a CNTN4 protein using the antibody or antigen-binding fragment thereof.

### Solution to Problem

The present invention provides an anti-CNTN4 antibody or an antigen-binding fragment thereof, which specifically binds to a CNTN4 protein. The antibody or antigen-binding fragment specifically binds to a CNTN4 protein, for example, a human or mouse CNTN4 protein, to neutralize an immune escape mechanism of CNTN4. Accordingly, the antibody or antigen-binding fragment of the present invention may increase the activity of T cells, such as CD4+ T cells or CD8+ T cells, which have been inhibited by CNTN4.

In an embodiment, the present invention provides an anti-CNTN4 antibody or an antigen-binding fragment thereof comprising:
a light chain CDR1 comprising an amino acid sequence of SEQ ID NO: 70;
a light chain CDR2 comprising an amino acid sequence of SEQ ID NO: 88;
a light chain CDR3 comprising an amino acid sequence of SEQ ID NO: 116;
a heavy chain CDR1 comprising an amino acid sequence of SEQ ID NO: 58;
a heavy chain CDR2 comprising an amino acid sequence of SEQ ID NO: 89; and
a heavy chain CDR3 comprising an amino acid sequence of SEQ ID NO: 115.

In an embodiment, the present invention provides an anti-CNTN4 antibody or an antigen-binding fragment thereof comprising:
a light chain variable region comprising an amino acid sequence of SEQ ID NO: 18; and
a heavy chain variable region comprising an amino acid sequence of SEQ ID NO: 2.

In an embodiment, the present invention provides an anti-CNTN4 antibody or an antigen-binding fragment thereof comprising:
a light chain variable region comprising an amino acid sequence of SEQ ID NO: 156; and
a heavy chain variable region comprising an amino acid sequence of SEQ ID NO: 159.

In an embodiment, the antigen-binding fragment of the present invention may be an antibody fragment selected from the group consisting of Fab, Fab', Fab'-SH, Fv, a single chain antibody scFv, a (Fab')2 fragment, a single domain antibody, a diabody (dAb), or a linear antibody, and the antibody may be a chimeric antibody or a humanized antibody.

In another embodiment, the present invention provides a nucleic acid molecule encoding the antibody or antigen-binding fragment thereof and a recombinant expression vector including the nucleic acid molecule.

In another embodiment, the present invention also provides a composition for preventing or treating cancer, the composition comprising the antibody or antigen-binding fragment thereof as an active ingredient. The pharmaceutical composition may be used in combination with an additional anticancer agent, such as an immune checkpoint inhibitor or a chemotherapeutic agent, or may be used in combination with radiation therapy.

In another embodiment, the present invention also provides a composition for analyzing or detecting a CNTN4 protein, the composition comprising the antibody or antigen binding fragment thereof.

In another embodiment, the present invention provides a use of an anti-CNTN4 antibody or an antigen-binding fragment thereof for preventing or treating cancer.

In another embodiment, the present invention provides a use of an anti-CNTN4 antibody or an antigen-binding fragment thereof for the preparation of a medicament for preventing or treating cancer.

In another embodiment, the present invention provides a method for preventing or treating cancer, the method comprising administering, to a subject, an effective amount of an anti-CNTN4 antibody or an antigen-binding fragment thereof.

In another embodiment, the present invention provides a method for preventing or treating cancer, the method comprising administering, to a subject, an effective amount of an anti-CNTN4 antibody or an antigen-binding fragment thereof, and an effective amount of an additional anticancer agent. As the additional anticancer agent, for example, an immune checkpoint inhibitor or a chemotherapeutic agent may be used.

### Advantageous Effects of Invention

The novel anti-CNTN4 antibody or antigen-binding fragment thereof of the present invention activates T-cells by blocking the immune escape mechanism of CNTN4, and thus can be effectively used to prevent or treat diseases caused by a decrease in T-cell activity, particularly, cancer. Therefore, when the anti-CNTN4 antibody of the present invention is used, an excellent anticancer effect may be exhibited with respect to cancer for which a therapeutic effect cannot be achieved with existing immunotherapy.

### Brief Description of Drawings

FIGS. 1 and 2 show amino acid sequences of light and heavy chain variable regions of scFv antibody fragments selected from phage display library. The solid and dashed line-marked sequences represent CDR sequences of each region.
FIG. 3 shows amino acid sequences of variable regions used to prepare an anti-CNTN4 humanized antibody.
FIG. 4 shows that antibodies A-01 and L1H2 of the present invention effectively neutralize CNTN4 proteins to proliferate T cells.

### Best Mode for Carrying out the Invention

### Anti-CNTN4 Antibody or Antigen-binding Fragment Thereof

The present invention provides an anti-CNTN4 antibody or an antigen-binding fragment thereof, which specifically binds to a CNTN4 protein.

In an embodiment, the anti-CNTN4 antibody or antigen-binding fragment thereof of the present invention comprises:
a light chain CDR1 comprising an amino acid sequence of SEQ ID NO: 70;
a light chain CDR2 comprising an amino acid sequence of SEQ ID NO: 88;
a light chain CDR3 comprising an amino acid sequence of SEQ ID NO: 116;
a heavy chain CDR1 comprising an amino acid sequence of SEQ ID NO: 58;
a heavy chain CDR2 comprising an amino acid sequence of SEQ ID NO: 89; and
a heavy chain CDR3 comprising an amino acid sequence of SEQ ID NO: 115.

Table 1 shows the CDR sequence of each of light and heavy chain variable regions for the anti-CNTN4 antibody or antigen-binding fragment thereof of the present invention.

**[Table 1]**

| Region | CDR1 | SEQ ID NO | CDR2 | SEQ ID NO | CDR3 | SEQ ID NO |
|---|---|---|---|---|---|---|
| S1 light chain variable region(VL) | SGGSS | 57 | DNTNRPS | 88 | GGYDSSIDA | 114 |
| S1 heavy chain variable region(VH) | SFNMF | 58 | EISGGGGSTWYA PAVKG | 89 | SADTWSYGA ATIDA | 115 |
| S2 light chain variable region(VL) | SGGGSSTY | 59 | NNNNRPS | 90 | GGYDGSTDV | 116 |
| S2 heavy chain variable region(VH) | SFNMF | 58 | EISGGGGSTWYA PAVKG | 89 | SADTWSYGA ATIDA | 115 |
| S3 light chain variable region(VL) | SGGSS | 57 | DNTNRPS | 88 | GGYDGSTDA | 117 |
| S3 heavy chain variable region(VH) | SFNMF | 58 | EISGGGGSTWYA PAVKG | 89 | SADTWSYGA ATIDA | 115 |
| S4 light chain variable region(VL) | SGGGSSY | 60 | QNTNRPS | 91 | GGYDGSTDV | 116 |
| S4 heavy chain variable region(VH) | SFNMF | 58 | EISGGGGSTWYA SAVKG | 92 | SADTWSYGA ATIDA | 115 |
| S6 light chain variable region(VL) | SGGSGN | 61 | NGNNRPS | 93 | GGYDGTIDA | 118 |
| S6 heavy chain variable region(VH) | SFNMF | 58 | EISGGGGSTWYA PAVKG | 89 | SADTWSYGA ATIDA | 115 |
| S7 light chain variable region(VL) | SGGSGS | 62 | DNTNRPS | 88 | GSYDSIEVI | 119 |
| S7 heavy chain variable region(VH) | SYGMA | 63 | GITSSGSSAGYG PAVKG | 94 | DGYIYSGADS DSIDA | 120 |
| S8 light chain variable region(VL) | SGGSGS | 62 | DNTNRPS | 88 | GSYDSIEVI | 119 |
| S8 heavy chain variable region(VH) | SYGMA | 63 | GITSSGSSAGYG PAVKG | 94 | DGYIYSGADS DSIDA | 120 |
| S9 light chain variable region(VL) | SGGSGS | 62 | DNTNRPS | 88 | GSYDSIEVI | 119 |
| S9 heavy chain variable region(VH) | SYGMA | 63 | GITSSGSSAGYG PAVKG | 94 | DGYIYSGADS DSIDA | 120 |
| S10 light chain variable region(VL) | SGGSGS | 62 | DNTNRPS | 88 | GSYDSIEVI | 119 |
| S10 heavy chain variable region(VH) | SYGMA | 63 | GITSSGSSAGYG PAVKG | 94 | DGYIYSGADS DSIDA | 120 |
| S11 light chain variable region(VL) | SGGSGS | 62 | DNTNRPS | 88 | GSYDSIEVI | 119 |
| S11 heavy chain variable region(VH) | SYGMA | 63 | GITSSGSSAGYG PAVKG | 94 | DGYIYSGADS DSIDA | 120 |
| S15 light chain variable region(VL) | SGGSGS | 62 | DNTNRPS | 88 | GSYDSIEVI | 119 |
| S15 heavy chain variable region(VH) | SYGMA | 63 | GITSSGSSAGYG PAVKG | 94 | DGYIYSGADS DSIDA | 120 |
| S24 light chain variable region(VL) | SGGSGS | 62 | DNTNRPS | 88 | GSYDSIEVI | 119 |
| S24 heavy chain variable region(VH) | SYGMA | 63 | GITSSGSSAGYG PAVKG | 94 | DGYIYSGGDS DSIDA | 121 |
| S30 light chain variable region(VL) | SGGSGS | 62 | DNTNRPS | 88 | GSYDSIEVI | 119 |
| S30 heavy chain variable region(VH) | SYGMA | 63 | GITSSGSSAGYG PAVKG | 94 | DGYIYSGADS DSIDA | 120 |
| S46 light chain variable region(VL) | SGGSGS | 62 | DNTNRPS | 88 | GSYDSIEVI | 119 |
| S46 heavy chain variable region(VH) | SYGMA | 63 | GITSSGSSAGYG PAVKG | 94 | DGYIYSGADS DSIDA | 120 |
| S83 light chain variable region(VL) | SGGGGYSGS YY | 64 | SNNQRPS | 95 | GSIDSSSNPG I | 122 |
| S83 heavy chain variable region(VH) | RYIMQ | 65 | SIRNGTTPKYGA AVKG | 96 | GDDGDCICG GCAASIDI | 123 |
| S96 light chain variable region(VL) | SGSSGSYG | 66 | ESNKRPS | 97 | GSADSSTTIA | 124 |
| S96 heavy chain variable region(VH) | TYSMQ | 67 | GISNSGSSTVYG PAVKG | 98 | STYGGSWYA AYSIDE | 125 |
| S99 light chain variable region(VL) | SGGASYAGN YY | 68 | NNNQRPS | 99 | GSDDSSAGY TGI | 126 |
| S99 heavy chain variable region(VH) | GYGMH | 69 | EISNTGSWTGYG AAVKG | 100 | VGGRGWCG RSGCADDID A | 127 |
| S129 light chain variable region(VL) | SGSSGS | 70 | DNTNRPS | 88 | GGYDGSTDV | 116 |
| S129 heavy chain variable region(VH) | SFNMF | 58 | EISGGGGSTWYA PAVKG | 89 | SADTWSYGA ATIDA | 115 |
| S130 light chain variable region(VL) | SGGGSSTY | 59 | NNNNRPS | 90 | GGYDGSTDV | 116 |
| S130 heavy chain variable region(VH) | SFNMF | 58 | EISGGGGSTWYA PAVKG | 89 | SADTWSYGA ATIDA | 115 |
| S131 light chain variable region(VL) | SGGGSSY | 60 | QNTNRPS | 91 | GGYDGSTDV | 116 |
| S131 heavy chain variable region(VH) | SFNMF | 58 | EISGGGGSTWYA PAVKG | 89 | SADTWSYGA ATIDA | 115 |
| S132 light chain variable region(VL) | SGGGSSTY | 59 | NNNNRPS | 90 | GGYDGSTDV | 116 |
| S132 heavy chain variable region(VH) | SFNMF | 58 | EISGGGGSTWYA PAVKG | 89 | SADTWSYGA ATIDA | 115 |
| S134 light chain variable region(VL) | SGGTYSYGN YY | 71 | NNNNRPS | 90 | GGYDGSTDV | 116 |
| S134 heavy chain variable region(VH) | SFNMF | 58 | EISGGGGSTWYA PAVKG | 89 | SADTWSYGA ATIDA | 115 |
| S135 light chain variable region(VL) | GGGSSSY | 72 | YNNRPS | 101 | GGYDGSTDV | 116 |
| S135 heavy chain variable region(VH) | SFNMF | 58 | EISGGGGSTWYA PAVKG | 89 | SADTWSYGA ATIDA | 115 |
| S137 light chain variable region(VL) | SGGSS | 57 | DNTNRPS | 88 | GGYDGSTDA | 117 |
| S137 heavy chain variable region(VH) | SFNMF | 58 | EISGGGGSTWYA PAVKG | 89 | SADTWSYGA ATIDA | 115 |
| S138 light chain variable region(VL) | SGGGSYAGS YY | 73 | DSTNRPS | 102 | GGYDGSTDV | 116 |
| S138 heavy chain variable region(VH) | SFNMF | 58 | EISGGGGSTWYA PAVKG | 89 | SADTWSYGA ATIDA | 115 |
| A13WB2 light chain variable region(VL) | SGGYSG | 74 | YNDKRPS | 103 | GSRDSSTYV DI | 128 |
| A13WB2 heavy chain variable region(VH) | SYGMA | 63 | GITSSGSSAGYG PAVKG | 94 | DGYIYSGADS DSIDA | 120 |
| A13WB4 light chain variable region(VL) | SGGSSDYG | 75 | YNNNRPS | 104 | GSRDSSSGY VDI | 129 |
| A13WB4 heavy chain variable region(VH) | SYGMA | 63 | GITSSGSSAGYG PAVKG | 94 | DGYIYSGADS DSIDA | 120 |
| A13WB7 light chain variable region(VL) | SGGGSSSYY N | 76 | YNDKRPS | 103 | GSWDSSTYS DI | 130 |
| A13WB7 heavy chain variable region(VH) | SYGMA | 63 | GITSSGSSAGYG PAVKG | 94 | DGYIYSGADS DSIDA | 120 |
| A13WB24 light chain variable region(VL) | SGGGNNN | 77 | YNDNRP | 105 | GGYDISYVDI | 131 |
| A13WB24 heavy chain variable region(VH) | SYGMA | 63 | GITSSGSSAGYG PAVKG | 94 | DGYIYSGADS DSIDA | 120 |
| A13WB25 light chain variable region(VL) | SGGGGS | 78 | YNDKRPS | 103 | GGYDGSTYA GI | 132 |
| A13WB25 heavy chain variable region(VH) | SYGMA | 63 | GITSSGSSAGYG PAVKG | 94 | DGYIYSGADS DSIDA | 120 |
| A13WB73 light chain variable region(VL) | SGGYSG | 74 | YNDKRPS | 103 | GSWDTSTNT PI | 133 |
| A13WB73 heavy chain variable region(VH) | SYGMA | 63 | GITSSGSSAGYG PAVKG | 94 | DGYIYSGADS DSIDA | 120 |
| A13SF1 light chain variable region(VL) | SGGGGS | 78 | NNNNRPS | 90 | GGYDSSTYV GT | 134 |
| A13SF1 heavy chain variable region(VH) | SYGMA | 63 | GITSSGSSAGYG PAVKG | 94 | DGYIYSGADS DSIDA | 120 |
| A13SF2 light chain variable region(VL) | SGGGGS | 78 | NNNNRPS | 90 | GGYDSSAGY AAL | 135 |
| A13SF2 heavy chain variable region(VH) | SYGMA | 63 | GITSSGSSAGYG PAVKG | 94 | DGYIYSGADS DSIDA | 120 |
| A13SF7 light chain variable region(VL) | GGSGS | 79 | DNTNRPS | 88 | GSADTNAAY AAI | 136 |
| A13SF7 heavy chain variable region(VH) | SYGMA | 63 | GITSSGSSAGYG PAVKG | 94 | DGYIYSGADS DSIDA | 120 |
| A13SF8 light chain variable region(VL) | SGSGN | 80 | YNDKRPS | 103 | GGYDGYSFA SI | 137 |
| A13SF8 heavy chain variable region(VH) | SYGMA | 63 | GITSSGSSAGYG PAVKG | 94 | DGYIYSGADS DSIDA | 120 |
| A13SF16 light chain variable region(VL) | SGSGGS | 81 | NNDNRPS | 106 | GGYDSSTYV DT | 138 |
| A13SF16 heavy chain variable region(VH) | SYGMA | 63 | GITSSGSSAGYG PAVKG | 94 | DGYIYSGADS DSIDA | 120 |
| A13SF19 light chain variable region(VL) | SGSSSGN | 82 | YNDERPS | 107 | GGYEGSTYV GI | 139 |
| A13SF19 heavy chain variable region(VH) | SYGMA | 63 | GITSSGSSAGYG PAVKG | 94 | DGYIYSGADS DSIDA | 120 |
| A13SF20 light chain variable region(VL) | SGGSGS | 62 | NSDKRPS | 108 | GGYDSSAI | 140 |
| A13SF20 heavy chain variable region(VH) | SYGMA | 63 | GITSSGSSAGYG PAVKG | 94 | DGYIYSGADS DSIDA | 120 |
| A13SF21 light chain variable region(VL) | SGSSGS | 70 | NNNNRPS | 90 | GGYDSSTYV GI | 141 |
| A13SF21 heavy chain variable region(VH) | SYGMA | 63 | GITSSGSSAGYG PAVKG | 94 | DGYIYSGADS DSIDA | 120 |
| A13SF74 light chain variable region(VL) | SGNSSGCSW | 83 | DNTNRPS | 88 | GIADSSGAGI | 142 |
| A13SF74 heavy chain variable region(VH) | SYAMQ | 84 | GISKGGGSTAYG AAVKG | 109 | TTDSNCCSS DSIDA | 143 |
| A35WB1 light chain variable region(VL) | SGGSGS | 62 | NNDQRPS | 110 | GGWDSNSG AI | 144 |
| A35WB1 heavy chain variable region(VH) | SYGMA | 63 | GITSSGSSAGYG PAVKG | 94 | DGYIYSGADS DSIDA | 120 |
| A35WB27 light chain variable region(VL) | SGGYSG | 74 | YNDKRPS | 103 | GSADSSTGY VAI | 145 |
| A35WB27 heavy chain variable region(VH) | SYGMA | 63 | GITSSGSSAGYG PAVKG | 94 | DGYIYSGADS DSIDA | 120 |
| A35SF3 light chain variable region(VL) | SGSSGS | 70 | DNTNRPS | 88 | GGYDSSTI | 146 |
| A35SF3 heavy chain variable region(VH) | SYGMA | 63 | GITSSGSSAGYG PAVKG | 94 | DGYIYSGADS DSIDA | 120 |
| A35SF5 light chain variable region(VL) | SGSSGSN | 85 | YNDNRPS | 111 | GAWESSSNP DI | 147 |
| A35SF5 heavy chain variable region(VH) | SYGMA | 63 | GITSSGSSAGYG PAVKG | 94 | DGYIYSGADS DSIDA | 120 |
| A35SF8 light chain variable region(VL) | SGGGGS | 78 | NNNNRPS | 90 | GGYDDSTYV GM | 148 |
| A35SF8 heavy chain variable region(VH) | SYGMA | 63 | GITSSGSSAGYG PAVKG | 94 | DGYIYSGADS DSIDA | 120 |
| A35SF13 light chain variable region(VL) | SGGGGS | 78 | NNDNRPS | 106 | GSADNNGYI AI | 149 |
| A35SF13 heavy chain variable region(VH) | SYGMA | 63 | GITSSGSSAGYG PAVKG | 94 | DGYIYSGADS DSIDA | 120 |
| A35SF14 light chain variable region(VL) | SGGGGS | 78 | NGNNRPS | 93 | GGYDSGTYS GI | 150 |
| A35SF14 heavy chain variable region(VH) | SYGMA | 63 | GITSSGSSAGYG PAVKG | 94 | DGYIYSGADS DSIDA | 120 |
| A35SF37 light chain variable region(VL) | SGGSNN | 86 | YNNKRPS | 112 | GGYDSSSNS DV | 151 |
| A35SF37 heavy chain variable region(VH) | SYGMA | 63 | GITSSGSSAGYG PAVKG | 94 | DGYIYSGADS DSIDA | 120 |
| A35SF38 light chain variable region(VL) | SGSSGS | 70 | NNNNRPS | 90 | GGYDSSTYV AI | 152 |
| A35SF38 heavy chain variable region(VH) | SYGMA | 63 | GITSSGSSAGYG PAVKG | 94 | DGYIYSGADS DSIDA | 120 |
| A35SF39 light chain variable region(VL) | SGGSGS | 62 | NNNNRPS | 90 | GGYDTDSPI | 153 |
| A35SF39 heavy chain variable region(VH) | SYGMA | 63 | GITSSGSSAGYG PAVKG | 94 | DGYIYSGADS DSIDA | 120 |
| A35SF75 light chain variable region(VL) | SGGGGS | 78 | NNTNRPS | 113 | GGYDSNTDY VG | 154 |
| A35SF75 heavy chain variable region(VH) | SYGMA | 63 | GITSSGSSAGYG PAVKG | 94 | DGYIYSGADS DSIDA | 120 |
| A35SF90 light chain variable region(VL) | SGSNNNN | 87 | NNNQRPS | 99 | GGYDSSIYFD I | 155 |
| A35SF90 heavy chain variable region(VH) | SYGMA | 63 | GITSSGSSAGYG PAVKG | 94 | DGYIYSGADS DSIDA | 120 |

In another specific embodiment, the anti-CNTN4 antibody or antigen-binding fragment thereof of the present invention may comprise each pair of light chain variable regions and heavy chain variable regions as set forth in Tables 4 and 5. For example, the antibody or antigen-binding fragment thereof of the present invention may comprise: a light chain variable region comprising an amino acid sequence of SEQ ID NO: 1; and a heavy chain variable region comprising an amino acid sequence of SEQ ID NO: 2 (S1). Similarly, the antibody or antigen-binding fragment thereof of the present invention may comprise: a light chain variable region comprising an amino acid sequence of SEQ ID NO: 3; and a heavy chain variable region comprising the amino acid sequence of SEQ ID NO: 2 (S2). Another antibody or antigen-binding fragment thereof may also comprise two pairs of light and heavy chain variable regions in the same manner.

In a preferred embodiment, the anti-CNTN4 antibody or antigen-binding fragment thereof of the present invention comprise: a light chain variable region comprising an amino acid sequence of SEQ ID NO: 18; and a heavy chain variable region comprising the amino acid sequence of SEQ ID NO: 2.

In another specific embodiment, the anti-CNTN4 antibody or antigen-binding fragment thereof of the present invention may comprise each pair of light chain variable regions and/or heavy chain variable regions as set forth in Table 12. For example, the antibody or antigen-binding fragment thereof of the present invention may comprise: a light chain variable region comprising the amino acid sequence of SEQ ID NO: 18; and a heavy chain variable region comprising an amino acid sequence of SEQ ID NO: 159 (LAH2). Similarly, the antibody or antigen-binding fragment thereof of the present invention may comprise: a light chain variable region comprising an amino acid sequence of SEQ ID NO: 156; and a heavy chain variable region comprising the amino acid sequence of SEQ ID NO: 159 (L1H2). Alternatively, the antibody or antigen-binding fragment thereof of the present invention maycomprise: a light chain variable region comprising an amino acid sequence of SEQ ID NO: 158; and a heavy chain variable region comprising the amino acid sequence of SEQ ID NO: 159 (L2H2).

In a preferred embodiment, the anti-CNTN4 antibody or antigen-binding fragment thereof of the present invention comprises: a light chain variable region comprising an amino acid sequence of SEQ ID NO: 156; and a heavy chain variable region comprising the amino acid sequence of SEQ ID NO: 159.

As used herein, that a light chain or heavy chain variable region comprisesa specific amino acid sequence means that the light chain or heavy chain variable region comprises the entire amino acid sequence, has the entire amino acid sequence, or consists of the amino acid sequence.

In one example, the antibody or antigen-binding fragment thereof comprises: a light chain CDR1 having the amino acid sequence of SEQ ID NO: 70; a light chain CDR2 having the amino acid sequence of SEQ ID NO: 88; a light chain CDR3 having the amino acid sequence of SEQ ID NO: 116; a heavy chain CDR1 having the amino acid sequence of SEQ ID NO: 58; a heavy chain CDR2 having the amino acid sequence of SEQ ID NO: 89; and a heavy chain CDR3 having the amino acid sequence of SEQ ID NO: 115.

In another example, the antibody or antigen binding fragment thereof comprises: a light chain CDR1 consisting essentially of the amino acid sequence of SEQ ID NO: 70; a light chain CDR2 consisting essentially of the amino acid sequence of SEQ ID NO: 88; a light chain CDR3 consisting essentially of the amino acid sequence of SEQ ID NO: 116; a heavy chain CDR1 consisting essentially of the amino acid sequence of SEQ ID NO: 58; a heavy chain CDR2 consisting essentially of the amino acid sequence of SEQ ID NO: 89; and a heavy chain CDR3 consisting essentially of the amino acid sequence of SEQ ID NO: 115.

In another example, the antibody or antigen binding fragment thereof comprises: a light chain CDR1 consisting of the amino acid sequence of SEQ ID NO: 70; a light chain CDR2 consisting of the amino acid sequence of SEQ ID NO: 88; a light chain CDR3 consisting of the amino acid sequence of SEQ ID NO: 116; a heavy chain CDR1 consisting of the amino acid sequence of SEQ ID NO: 58; a heavy chain CDR2 consisting of the amino acid sequence of SEQ ID NO: 89; and a heavy chain CDR3 consisting of the amino acid sequence of SEQ ID NO: 115.

In another example, the antibody or antigen binding fragment thereof comprises a light chain variable region having the amino acid sequence of SEQ ID NO: 18 and a heavy chain variable region having the amino acid sequence of SEQ ID NO: 2, or comprises a light chain variable region having the amino acid sequence of SEQ ID NO: 156 and a heavy chain variable region having the amino acid sequence of SEQ ID NO: 159.

In another example, the antibody or antigen-binding fragment thereof comprises a light chain variable region consisting essentially of the amino acid sequence of SEQ ID NO: 18 and a heavy chain variable region consisting essentially of the amino acid sequence of SEQ ID NO: 2, or comprises a light chain variable region consisting essentially of the amino acid sequence of SEQ ID NO: 156 and a heavy chain variable region consisting essentially of the amino acid sequence of SEQ ID NO: 159.

As another example, the antibody or antigen-binding fragment thereof comprises a light chain variable region consisting of the amino acid sequence of SEQ ID NO: 18 and a heavy chain variable region consisting of the amino acid sequence of SEQ ID NO: 2, or comprises a light chain variable region consisting of the amino acid sequence of SEQ ID NO: 156 and a heavy chain variable region consisting of the amino acid sequence of SEQ ID NO: 159.

As used herein, the terms "comprising," "comprise," or modified words thereof refer to an open meaning. As an example, an antibody or antigen-binding fragment thereof comprising the amino acid sequence listed may include additional amino acid sequences not listed, whether essential or not.

As used herein, the term "consisting essentially of" or a modified phrase thereof includes any of the mentioned elements, and permits the presence of elements that do not substantially affect the basic and novel or functional characteristics of the embodiment. As an example, an antibody or antigen-binding fragment thereof consisting essentially of the amino acid sequence listed may include a substitution of one or more amino acid residues that do not substantially affect the characteristics of the antibody or fragment thereof.

As used herein, the term "consisting of" or a modified phrase thereof refers to the case where respective components as described herein do not permit any element that is not recited or listed in that description of the embodiment.

As used herein, the term "complementarity determining region" (CDR; CDR1, CDR2, and CDR3) refers to the amino acid residues within the antibody variable regions, which are necessary for antigen binding. Each variable region typically has three CDR regions identified as CDR1, CDR2, and CDR3.

In another preferred embodiment, the antibody or antigen-binding fragment thereof of the present invention may include a sequence in which the framework region of scFv is fully or partially humanized. A partially humanized sequence may be at least about 60%, at least about 70%, at least about 80% humanized as compared with a fully humanized sequence, and some of the fully humanized sequence (e.g., 1, 2, 3, 4 or 5, etc.) may be back-mutated. The antibody or antigen-binding fragment thereof comprising the humanized sequence may have an equivalent binding ability to CNTN-4 or not have substantially large difference in binding ability to CNTN-4, even when compared with a non-humanized antibody or antigen-binding fragment thereof.

The term "antibody" as used herein refers to an immunoglobulin molecule capable of specifically binding to a target, such as a carbohydrate, polynucleotide, lipid, polypeptide, protein, etc., through at least one antigen recognition site, located in the variable region of the immunoglobulin molecule. The term "antibody" herein encompasses not only an intact polyclonal or monoclonal antibody, but also an antigen-binding fragment thereof, and a fusion protein including an antibody fragment, and any other modified configuration of the immunoglobulin molecule that includes an antigen recognition site.

An antibody includes five classes of immunoglobulin (Ig)M, IgD, IgG, IgA, and IgE, comprising heavy chains made from the heavy chain constant region genes µ, δ, γ, α, and ε, respectively. The light chain and the heavy chain of the antibody are divided into variable regions having different amino acid sequences for each antibody and constant regions having the same amino acid sequence, and the heavy chain constant regions comprise CH1, H (hinge), CH2, and CH3 domains. Each domain consists of two β-sheets and an intramolecular disulfide bond is linked therebetween.

An antibody comprising a light chain variable region consisting of the amino acid sequence of SEQ ID NO: 18; and a heavy chain variable region consisting of the amino acid sequence of SEQ ID NO: 2 is referred to as "A-01" herein.

In addition, an antibody comprising a light chain variable region consisting of the amino acid sequence of SEQ ID NO: 156; and a heavy chain variable region consisting of the amino acid sequence of SEQ ID NO: 159 is referred to as "L1H2" herein.

The antibody of the present invention may be a chimeric antibody, a humanized antibody, or a human antibody.

As used herein, the term "chimeric antibody" refers to antibodies in which the variable region sequences are derived from one species and the constant region sequences are derived from another species, such as an antibody in which the variable region sequences are derived from a mouse or chicken antibody and the constant region sequences are derived from a human antibody.

As used herein, the term "humanized antibody" refers to antibodies in which CDR sequences derived from the germline of another mammalian species, such as, a mouse or chicken, have been grafted onto human framework sequences. The framework sequences may further be remodified via back mutation, for example.

As used herein, the term "human antibody" refers to antibodies in which both frameworks and CDR regions comprise variable regions derived from human immunoglobulin sequences. The constant regions of antibodies are also derived from human immunoglobulin sequences.

As used herein, the term "antigen-binding fragment" or "antibody fragment" refers to antigen-binding fragments and analogues of an antibody, typically comprising at least a portion of the antigen binding or variable regions (e.g. one or more CDRs) of the parental antibody. An antibody fragment retains at least some of the binding specificity of the parent antibody. Examples of antibody fragments include, but are not limited to, Fab, Fab', Fab'-SH, Fv, F(ab')2 fragments, single-chain antibodies scFv, single domain antibodies, diabodies (dAbs), or linear antibodies.

Specifically, the Fab fragment refers to a monovalent fragment composed of VL, VH, CL, and CH1 domains.

The Fab' fragment differs from the Fab fragment in that several residues are added at the carboxyl terminus of the CH1 domain comprising at least one cysteine from the antibody hinge region.

The Fab'-SH refers to a Fab' in which the cysteine residue of the constant domain has a free thiol group.

The F(ab')2 antibody fragment is generated as a pair of Fab' fragments via a hinge cysteine between the Fab' fragments.

The Fv is a minimum antibody fragment containing a complete antigen-recognition site and - binding site. This fragment consists of a dimer of one heavy chain variable region and one light chain variable region in tight, non-covalent association. From the folding of these two regions emanate six hypervariable loops (three loops each from the heavy and light chain) that contribute the amino acid residues for antigen-binding and confer antigen binding specificity to the antibody. However, even a single variable region has the ability to recognize and bind antigen, although at a lower affinity than the entire binding site.

The single chain antibody scFv is an antibody fragment that comprises VH and VL antibody domains linked into a single polypeptide chain. Preferably, the scFv polypeptide further comprises a polypeptide linker between the VH and VL domains that enables the scFv to form the desired structure for antigen binding. The scFv polypeptide herein is also referred to scFv antibody fragment, antigen-binding fragment scFv, scFv antibody, antibody scFv, or simply scFv.

The diabodies refer to small antibody fragments prepared by constructing scFv fragments using short linkers (about 5-10 residues) between the VH and VL domains such that inter-chain but not intra-chain pairing of the V domains is achieved, resulting in a bivalent fragment, i.e., a fragment having two antigen-binding sites. Bispecific diabodies are heterodimers consisting of two "crossover" scFv fragments in which the VH and VL domains of the two antibodies are present on different polypeptide chains.

The anti-CNTN4 antibody or antigen-binding fragment thereof of the present invention may specifically bind to an CNTN4 protein, preferably a human or mouse CNTN4 protein.

As used herein, the term "specifically binds to" or "specific for" refers to measurable and reproducible interactions such as binding between a target and an antibody, which is determinative of the presence of the target in the presence of a heterogeneous population of molecules including biological molecules. For example, an antibody that specifically binds to a specific target (e.g., an epitope) is an antibody that binds this target with greater affinity, binding ability, more readily, and/or with greater duration than it binds to other targets.

As used herein, the term "specifically binding to a human CNTN4 protein" may refer to an antibody that binds to a human CNTN4 protein with a dissociation constant (Kd) of 5 ×10⁻⁷ M or less, or preferably 1 ×10⁻⁷ M or less. Accordingly, the anti-CNTN4 antibody or antigen binding fragment thereof of the present invention may bind to human CNTN4 protein with a dissociation constant (Kd) of 5 × 10⁻⁷ M or less, preferably 1 ×10⁻⁷ M or less.

As used herein, the term "Kd" refers to an equilibrium dissociation constant of a particular antibody-antigen interaction, wherein the constant has a unit of M. The Kd values for antibodies can be determined using methods widely established in the art. A preferred method for determining the Kd value of an antibody is by using surface plasmon resonance (SRP), preferably using a biosensor system such as a Biacore^{®} system.

In an embodiment, the anti-CNTN4 antibody or antigen-binding fragment thereof of the present invention is single specific and specifically binds to a single epitope, i.e., a CNTN4 protein.

In another embodiment, the anti-CNTN4 antibody or antigen-binding fragment thereof of the present invention is a multi-specific antibody molecule, such as a bi-specific or tri-specific antibody molecule. The multi-specific antibody molecule includes a plurality of variable regions, each variable region having binding specificity to a different epitope. In an embodiment, the first variable region of the bispecific antibody molecule has a first binding specificity to a first epitope, e.g., a CNTN4 protein, and the second variable region has a second binding specificity to a second epitope, e.g., a target protein other than the CNTN4 protein (e.g., including but not limited to CTLA-4, PD-1, or PD-L1). In one specific embodiment, the bispecific antibody molecule specifically binds to CNTN4; and any one of CTLA-4, PD-1 or PD-L1. In another embodiment, any combination of the above molecules can be prepared with a multi-specific antibody molecule, e.g., a tri-specific antibody including a first binding specificity to CNTN4, and second and third binding specificities to at least two of CTLA-4, PD-1 or PD-L1. The multi-specific antibody molecule of the present invention may be prepared using standard molecular biological techniques known to those skilled in the art (e.g., recombinant DNA and protein expression technology).

In another embodiment, the anti-CNTN4 antibody or antigen-binding fragment thereof of the present invention may form an antibody-drug conjugate (ADC). As used herein, the term "antibody-drug conjugate" or "ADC" may be represented by the formula M-[L-D]ₙ, wherein M represents an antibody molecule, i.e., an anti-CNTN4 antibody of the present invention or an antigen-binding fragment thereof, L is an optional linker or linker unit, D is a suitable drug or prodrug, and n is an integer from about 1 to about 20. The drug included in the ADC may be appropriately selected according to therapeutic or diagnostic use, as long as the drug does not interfere with specific binding of the antibody of the present invention. In an embodiment, the drug includes, but is not limited to, a cytotoxic agent (e.g., a chemotherapeutic agent), a prodrug converting enzyme, a radioactive isotope or compound, or a toxin. Drugs and linkers that may be included in the ADC and preparation methods thereof may be according to methods known in the art.

In another embodiment, the anti-CNTN4 antibody or antigen-binding fragment thereof of the present invention binds to a CNTN4 protein (e.g., a human or mouse CNTN4 protein) with an EC50 of 5 nM or less, preferably 3 nM or less, more preferably 2 nM, and even more preferably 1 nM or less as determined by ELISA assay.

As used herein, the term "EC50" is a term related to an in vitro or in vivo assay using an antibody, which refers to the concentration of an antibody that induces 50% of the maximum response, i.e., an intermediate response between the maximum response and baseline.

### Nucleic Acid Molecule and Vector

Another aspect of the present invention relates to a nucleic acid molecule encoding the anti-CNTN4 antibody or antigen-binding fragment thereof of the present invention.

The nucleic acid may be present in whole cells, in a cell lysate, or in a specifically purified or substantially pure form. A nucleic acid is "isolated" or "rendered substantially pure" when purified away from other cellular components or other contaminants, e.g., other cellular nucleic acids or proteins, by standard techniques, including alkaline/SDS treatment, CsCl banding, column chromatography, agarose gel electrophoresis and others well known in the art.

The nucleic acid of the present invention may be, for example, DNA or RNA and may or may not contain intronic sequences. In a preferred embodiment, the nucleic acid is a cDNA molecule.

In an embodiment, the nucleic acid molecule of the present invention encodes the light chain region, the heavy chain region, or both the light chain and the heavy chain regions of the anti-CNTN4 antibody or antigen-binding fragment thereof of the present invention, and preferably encodes the light chain variable region, the heavy chain variable region, or both the light chain and the heavy chain variable regions. In an embodiment, the nucleic acid molecule of the present invention encodes a light chain variable region comprising the amino acid sequence of SEQ ID NO: 18 or SEQ ID NO: 156, or encodes a heavy chain variable region comprising the amino acid sequence of SEQ ID NO: 2 or SEQ ID NO: 159. Alternatively, the nucleic acid molecule of the present invention encodes antibody A-01 or antibody L1H2.

Once DNA fragments encoding the VL and/or VH regions are obtained, such DNA fragments can be further manipulated by standard recombinant DNA techniques, for example, to thus convert the variable region genes to full-length antibody chain genes, to Fab fragment genes or to a scFv gene. In these manipulations, a VL- or VH-encoding DNA fragment is operatively linked to another DNA fragment encoding another protein, for example an antibody constant region, such as hIgG1 Fc region (hFc) or hCκ region, or a flexible linker. As used herein, the term "operatively linked" means that the two DNA fragments are joined such that the amino acid sequences encoded by the two DNA fragments remain in-frame.

The isolated DNA encoding the VH region can be converted to a full-length heavy chain gene by operatively binding the VH-encoding DNA to another DNA molecule encoding heavy chain constant regions (CH1, CH2 and CH3). The heavy chain constant region may be an IgG1, IgG2, IgG3, IgG4, IgA, IgE, IgM, or IgD constant region.

For a Fab fragment heavy chain gene, the VH-encoding DNA can be operatively linked to another DNA molecule encoding only the heavy chain CH1 constant region.

To create a scFv gene, the VL- and VH-encoding DNA fragments are operatively linked to another fragment encoding a flexible linker, e.g., encoding the amino acid sequence (Gly4-Ser)3, such that the VL and VH sequences can be expressed as a contiguous single-chain protein, with the VL and VH regions joined by the flexible linker.

The nucleic acid sequences of the present invention, such as RNA or DNA, may be isolated from a variety of sources, genetically engineered, amplified, and/or expressed recombinantly. Any recombinant expression system can be used, including, in addition to bacterial, e.g., yeast, insect or mammalian systems. For example, the manipulation of nucleic acids, such as subcloning into expression vectors, labeling probes, sequencing, and hybridization may be performed as known in the art.

Accordingly, the present invention provides a recombinant expression vector including the nucleic acid molecule.

As used herein, the term "vector" refers to a DNA molecule capable of self-replication in prokaryotic and/or eukaryotic cells and is used interchangeably with a recombinant vector, a cloning vector, or an expression vector, which is generally used as a carrier for delivering genes or DNA fragments to cells and the like. The vectors generally include, but are not limited to, an origin of replication that can be replicated in prokaryotic and/or eukaryotic cells, a selection marker gene that can confer resistance to a particular conditions/substances such as antibiotic degrading enzymes, a promoter capable of transcription of genes in eukaryotic or prokaryotic cells, and translatable sequences.

One type of vector is a "plasmid," which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors).

The present invention also provides a host cell including the vector. Host cells include, but are not limited to, cells of mammalian, plant, insect, fungal, or bacterial origin. Bacterial cells include cells from Gram-positive bacteria or Gram-negative bacteria such as several species of the genera *Escherichia*, such as E. coli, and *Pseudomonas.* In the group of fungal cells, preferably yeast cells are used. Expression in yeast may be achieved by using yeast strains such as, *inter alia*, *Pichia pastoris*, *Saccharomyces cerevisiae* and *Hansenula polymorpha.* In addition, insect cells, such as cells from *Drosophila* and Sf9 may be used as host cells.

In addition, expression systems using mammalian cells, such as Chinese Hamster Ovary (CHO) cells, simian COS cells, BHK cells, NSO cells or Bowes melanoma cells may be used. Since the antibody or antigen-binding fragment thereof of the present invention will ultimately be administered to a human, a complete human expression system may be particularly preferred. In this case, the host cells can be human cells, such as HeLa, 911, AT1080, A549, 293 and HEK293 cells.

### Preparation of Antibody or Antigen-Binding Fragment thereof

The antibody or antigen-binding fragment thereof of the present invention may be prepared according to conventionally known methods.

In an embodiment, an antibody of the invention, such as a monoclonal antibody, can be prepared by injecting a CNTN4 antigen into a test subject (e.g., a mouse) according to methods known in the art and then isolating a hybridoma that expresses the antibody having a sequence of interest or functional characteristics.

DNA encoding the monoclonal antibodies is readily isolated and sequenced using conventional procedures (e.g., by using oligonucleotide probes that are capable of binding specifically to genes encoding the heavy and light chains of monoclonal antibodies). Hybridoma cells are provided as a preferred source of such DNA. Once isolated, the DNA may be placed into expression vectors, which are then transfected into host cells such as E. coli cells, simian COS cells, CHO cells, or myeloma cells that do not otherwise produce immunoglobulin protein, to obtain the synthesis of monoclonal antibodies in the recombinant host cells.

In another embodiment, the antibody or antigen binding fragment thereof of the present invention can be prepared using antibody display techniques, such as phage library techniques.

The phage library of the antibody is cloned into a form in which the heavy and light chain variable region genes of the human antibody are fused to the phage surface protein (pIII) in a phagemid vector to be expressed in E. coli, and then M13 helper phage is infected to prepare an antibody library in which antibody fragments (scFv or Fab) having sequences of various combinations of heavy and light chain variable regions are displayed on the surface of the phage. From this library, fragments of an antibody binding to a specific antigen are isolated using a panning method, the isolated antibody fragments are characterized, and are then converted into whole IgG form and mass-expressed in animal cells, thereby producing specific human monoclonal antibodies.

As used herein, the term "phagemid vector" refers to a plasmid DNA having a phage origin of replication, which typically has an antibiotic resistance gene as a selection marker. A phagemid vector that is used in phage display includes the gIII gene of M13 phage or a portion thereof, and the ScFv gene is ligated to the 5' end of the gIII gene and expressed through a transformant.

A "helper phage" is a phage that provides necessary genetic information so that phagemid is packaged into phage particles. Since phagemid includes the gIII gene of phage or a portion thereof, a host cell (transformant) transformed with the phagemid is infected with the helper phage to provide the remaining phage gene. The helper phages include M13K07 or VCSM13, and mostly include an antibiotic resistance gene such as a kanamycin resistance gene so that a transformant infected with the helper phage can be selected. In addition, the packaging signal is defective, and thus the phagemid gene rather than the helper phage gene is selectively packaged into phage particles.

### Uses and Methods

The antibody or antigen-binding fragment thereof of the present invention recovers an immune response inhibited by the binding of the CNTN4 protein. It has been found that the CNTN4 protein inhibits the proliferation of T cells, particularly, CD4+ T cells and CD8+ T cells. The antibody or antigen-binding fragment thereof of the present invention may be used to treat diseases related to immunosuppression by specifically binding to the CNTN4 protein to thus increase T cell activity, particularly the activity of CD4+ T cells or CD8+ T cells.

In an embodiment, the antibody or antigen-binding fragment thereof of the present invention increases T cell activity. Accordingly, the antibody or antigen-binding fragment thereof of the present invention may activate T cells, particularly CD4+ T cells or CD8+ T cells. In an embodiment, the antibody or antigen-binding fragment thereof of the present invention may increase the proliferation of T cells that have been inhibited by CNTN4. The antibody or antigen-binding fragment thereof of the present invention has excellent ability to neutralize CNTN4.

Accordingly, the present invention relates to the induction of T-cell activation by using an anti-CNTN4 antibody or an antigen-binding fragment thereof. In an embodiment, the present invention provides a method for inducing or enhancing T cell activation, the method comprising administering, to a subject, an effective amount of an anti-CNTN4 antibody or an antigen-binding fragment thereof. In another embodiment, the present invention provides a use of an anti-CNTN4 antibody or an antigen-binding fragment thereof for inducing or enhancing T cell activation. In another embodiment, the present invention provides a pharmaceutical composition for inducing or enhancing T cell activation, the composition comprising an anti-CNTN4 antibody or an antigen-binding fragment thereof.

Accordingly, the present invention relates to the prevention, amelioration, or treatment of immunosuppression-related diseases by using an anti-CNTN4 antibody or an antigen-binding fragment thereof.

In an embodiment, the present invention provides a method for preventing, ameliorating or treating immunosuppression-related diseases, the method comprising administering, to a subject, an effective amount of an anti-CNTN4 antibody or an antigen-binding fragment thereof.

In another embodiment, the present invention provides a use of an anti-CNTN4 antibody or an antigen-binding fragment thereof for preventing, ameliorating or treating immunosuppression-related diseases.

In another embodiment, the present invention provides a pharmaceutical composition for preventing, ameliorating, or treating immunosuppression-related diseases, the composition comprising an anti-CNTN4 antibody or an antigen-binding fragment thereof. As used herein, the term "subject" is meant to include both human and non-human animals. Non-human animals include all vertebrates, e.g., mammals and non-mammals, including non-human primates, sheep, dogs, cats, cattle, horses, chickens, amphibians and reptiles, but, for example, non-human primates, sheep, dogs, cats, cattle, and horses are preferred. A preferred subject is a human in need of immune response activation or enhancement.

Preferably, the anti-CNTN4 antibody or antigen-binding fragment thereof of the present invention blocks the binding of the CNTN4 protein, thereby activating T cells and/or enhancing an immune response to cancer cells in a cancer patient, and thus may inhibit the growth of cancer cells *in vivo*, and therefore may be effectively used to prevent, ameliorate, or treat cancer.

In another embodiment, the present invention provides a use of an anti-CNTN4 antibody or an antigen-binding fragment thereof for preventing or treating cancer.

In another embodiment, the present invention provides a use of an anti-CNTN4 antibody or an antigen-binding fragment thereof for the preparation of a medicament for preventing or treating cancer.

In another embodiment, the present invention provides a method for preventing or treating cancer, the method comprising administering, to a subject, an effective amount of an anti-CNTN4 antibody or an antigen-binding fragment thereof.

In another embodiment, the present invention provides a method for preventing or treating cancer, the method comprising administering, to a subject, an effective amount of an anti-CNTN4 antibody or an antigen-binding fragment thereof, and an effective amount of an additional anticancer agent.

The additional anticancer agent may be an immune checkpoint inhibitor or a chemotherapeutic agent, and the immune checkpoint inhibitor may be one or more selected from the group consisting of anti-CTLA-4 antibodies, anti-PD-1 antibodies, and anti-PD-L1 antibodies.

The effective amount of the anti-CNTN4 antibody or antigen-binding fragment thereof, and the effective amount of the additional anticancer agent may be simultaneously administered to a subject as a single formulation, or may be simultaneously or sequentially administered to a subject as a separate formulation.

Preferred cancers of which growth may be inhibited when the antibodies of the present invention are used include cancers typically responsive to immunotherapy. For example, cancers in the present invention include, but are not limited to, melanoma (e.g., metastatic malignant melanoma), kidney cancer (e.g., clear cell carcinoma), prostate cancer (e.g., hormone refractory prostate adenocarcinoma), breast cancer, colorectal cancer, rectal cancer, colon cancer, and lung cancer (e.g., non-small cell lung cancer). In addition, a subject to be treated of the present invention includes refractory or recurrent malignancies of which growth may be inhibited when the antibodies of the present invention are used.

Examples of other cancers that may be treated using the methods of the present invention include bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, bladder cancer, kidney cancer, ureteral cancer, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, and combinations of these cancers.

In another embodiment, the cancers may be cancers that express CNTN4.

In another embodiment, the cancers may be refractory or resistant to conventional immune checkpoint inhibitors (e.g., resistant to PD-1 pathway inhibitors, PD-L1 pathway inhibitors, or CTLA-4 pathway inhibitors).

The antibody or antigen-binding fragment of the present invention may be used alone or in combination with other anticancer therapies. Other anticancer therapies may include, for example, standard cancer therapies (e.g., chemotherapies, radiotherapies, or surgery); or other anticancer agents, such as cytotoxic, cytostatic, anti-angiogenic or antimetabolite agents, tumor targeted agents, immune stimulating or immune modulating agents, or antibodies conjugated to cytotoxic, cytostatic, or other toxic agents, immune checkpoint inhibitors, and the like.

Preferably, the antibody or antigen-binding fragment of the present invention may be used in combination with other anticancer agents such as immune checkpoint inhibitors, chemotherapeutic agents, or radiotherapies. The immune checkpoint inhibitors may be, for example, anti-CTLA-4 antibodies (e.g., ipilimumab), anti-PD-1 antibodies (e.g., pembrolizumab, nivolumab), or anti-PD-L1 antibodies (e.g., atezolizumab, avelumab, durvalumab). The chemotherapeutic agents include, but are not limited to, alkylating agents, antimetabolites, kinase inhibitors, spindle poison plant alkaloids, cytotoxic/antitumor antibiotics, topoisomerase inhibitors, photosensitizers, anti-estrogens and selective estrogen receptor modulators (SERMs), anti-progesterones, estrogen receptor down-regulators (ERDs), estrogen receptor antagonists, luteinizing hormone-releasing hormone agonists, anti-androgens, aromatase inhibitors, EGFR inhibitors, VEGF inhibitors, anti-sense oligonucleotides that inhibit expression of genes implicated in abnormal cell proliferation or tumor growth. Specific examples of chemotherapeutic agents of the present invention include gemcitabine, vinorelbine, etoposide (VP-16), platinum analogs such as cisplatin or carboplatin, taxoids such as paclitaxel, albumin-binding paclitaxel, and doxetaxel, or the like. Other cancer therapeutic agents include probiotics exhibiting anticancer effects, such as *Lactococcus lactis* GEN3013 strain (KCTC13426BP), *Lactococcus lactis* GEN3033 strain (KCTC13684BP), *Bifidobacterium bifidum* MG731 strain (KCTC13452BP), and the like.

When the antibody or antigen-binding fragment of the present invention is used in combination with other anticancer agents, these may be administered separately or may be applied in the form of a combination product in which a plurality of active ingredients are present in a single pharmaceutical formulation. When these are administered as separate formulations, the two formulations may be administered sequentially or simultaneously. For simultaneous administration, these are given together to the patient. For sequential administration, these may be given at a time interval that is not long, for example, these formulations may be administered to a patient within a period of 12 hours or less, or 6 hours or less.

In an embodiment, the present invention provides a method of preventing, ameliorating, or treating immunosuppression-related diseases, such as cancer, the method comprising administering, to a subject, an effective amount of an anti-CNTN4 antibody or an antigen-binding fragment thereof in combination with an additional anticancer agent. The embodiment includes not only simultaneously administering a single composition containing an anti-CNTN4 antibody or an antigen-binding fragment together with an additional anticancer agent to a patient in need thereof, but also simultaneously or sequentially administering compositions separately and respectively containing the anti-CNTN4 antibody or antigen-binding fragment and the additional anticancer agent to a patient in need thereof.

In another embodiment, the present invention provides a use of an anti-CNTN4 antibody or antigen-binding fragment thereof for using in combination with an additional anticancer agent for preventing, ameliorating, or treating immunosuppression-related diseases, such as cancer.

In another embodiment, the present invention provides a pharmaceutical composition or combination for preventing, ameliorating, or treating immunosuppression-related diseases, such as cancer, the composition or combination comprising an anti-CNTN4 antibody or an antigen-binding fragment thereof, and an additional anticancer agent. The pharmaceutical composition or combination herein comprising an anti-CNTN4 antibody or an antigen-binding fragment thereof, and an additional anticancer agent, include not only the case where the two components are physically present together in the form of a single formulation, but also the case where the two components are administered simultaneously or sequentially in separate formulations, wherein the two drugs may be provided separately or together in a single kit. Accordingly, the present invention provides a kit for preventing, ameliorating, or treating immunosuppression-related diseases, such as cancer, the kit comprising an anti-CNTN4 antibody or an antigen-binding fragment thereof and an additional anticancer agent.

The additional anticancer agent may preferably be immune checkpoint inhibitors, more preferably, anti-CTLA-4 antibodies (e.g., ipilimumab), anti-PD-1 antibodies (e.g., pembrolizumab and nivolumab), or anti-PD-L1 antibodies (e.g., atezolizumab, avelumab, and durvalumab).

Another preferred additional anticancer agent may include chemotherapeutic agents such as gemcitabine, vinorelbin, etoposide (VP-16), platinum analogs such as cisplatin or carboplatin, taxoids such as paclitaxel, albumin-binding paclitaxel, or doxetaxel.

Another preferred additional anticancer therapy used with the antibody or antigen-binding fragment thereof of the present invention may include radiotherapy.

The present invention also provides a method for detecting the presence of the CNTN4 protein in a sample or measuring the amount of the anti-CNTN4 antibodies by using the anti-CNTN4 antibody or antigen-binding fragment thereof as an active ingredient. The method comprises bringing the antibody or antigen-binding fragment thereof into contact with a sample and a control sample under the conditions under which the antibody or antigen-binding fragment thereof can bind to the CNTN4 protein to form a complex. Then, whether the complex has been formed is detected, wherein a difference in the degree of complex formation between the samples compared to the control sample is evidence that human blood antigens are present in the sample (e.g., blood).

Accordingly, the present invention provides a composition for diagnosing cancer, the composition comprising the anti-CNTN4 antibody or antigen-binding fragment thereof.

### Pharmaceutical Composition

The present invention provides a pharmaceutical composition comprising an anti-CNTN4 antibody or an antigen-binding fragment thereof. The composition may contain inactive ingredients, that is, pharmaceutically acceptable excipients (see Handbook of Pharmaceutical Excipients, etc.). Compositions of therapeutic and diagnostic formulations may be prepared in the form of, for example, freeze-dried powder, slurry, aqueous solution or suspension by mixing the formulations with physiologically acceptable carriers, excipients, or stabilizers.

Suitable route of administration includes parenteral administration, such as intramuscular, intravenous, or subcutaneous administration. Administration of the antibodies used in the pharmaceutical composition of the present invention or used to practice the methods of the present invention may be carried out by a variety of conventional methods such as topical application, or intradermal, subcutaneous, intraperitoneal, parenteral, intraarterial, or intravenous injection. In an embodiment, the antibody of the present invention is administered intravenously or subcutaneously.

Hereinafter, the present invention will be described in more detail with reference to Examples. It will be apparent to those skilled in the art that these Examples are only intended to describe the present invention in more detail, and the scope of the present invention is not limited by these Examples according to the subject matter of the present invention.

### Modes for the Invention

Hereinafter, the present invention will be described in more detail with reference to examples. The following examples are merely illustrative of the present invention, and the scope of the present invention is not limited thereto.

### Example 1: CNTN4 Specific Binding scFv Antibody Selection

### 1.1 scFv Antibody Library Preparation and Antibody Selection

### (1) Preparation of Chicken scFv Antibody Library

Fifteen chickens were divided into three groups of five each, and a first group was inoculated with mouse CNTN4, a second group with human CNTN4, and a third group with mouse CNTN4 and human CNTN4 alternately over a total of four times to generate antibodies. The amount of antigen inoculated was 20 µg/chicken for each group at a first time, 10 µg/chicken at a second time after 3 weeks, 10 µg/chicken at a third time after 2 weeks, and 10 µg/chicken at a fourth time after 2 weeks, and each group became immune with those amounts. Before antigen inoculation at each time, blood was collected with a syringe to confirm whether or not the antibodies were generated through ELISA which binds serum to the antigen. After the third inoculation, a total of four chickens showing reactivity in both human CNTN4 and mouse CNTN4 in ELISA were selected, and specifically, 3^{rd} chicken in the first group (hereinafter, referred to as 1-3), 1-4, 3-2, and 3-5 were selected. After sacrifice, the blood, spleen, bone marrow, and bursa fabricius were extracted, and total RNA was extracted. The main RNA, 28S rRNA, was well identified, and cDNA was synthesized by using SuperScript^{™} IV First-Strand Synthesis System (Invitrogen, # 18091050) using, as a template, RNA extracted for PCR of the immune antibody library. Through PCR, scFv fragment in which the light chain and heavy chain variable regions of the antibody were linked with a linker was secured, cloning was performed on pComb3X which is phagemid vector by using restriction enzyme Sfi I for phage display, and electroporation was performed on *E.coli* ER2738 having F' pili capable of infection with M13 helper phage. The complexity of the scFv library is confirmed as shown in Table 2.

**[Table 2]**

| Anti-chicken CNTN4 scFv library (Number of the binding clones) | | | | |
|---|---|---|---|---|
| Chicken number | 1-3 | 1-4 | 3-2 | 3-5 |
| Whole blood | 1.5 x 10⁸ | 4.0 x 10⁸ | 1.4 x 10⁹ | 9.3 x 10⁸ |
| Bone marrow | - | 4.7 x 10⁸ | 9.3 x 10⁸ | 1.5 x 10⁹ |
| Spleen | 3.5 x 10⁸ | - | - | 9.2 x 10⁸ |
| Bursa Fabricius | 1.2 x 10⁹ | - | - | 1.3 x 10⁹ |

### (2) scFv Antibody Selection

For each of chicken numbers 1-3, 1-4, 3-2, and 3-5 in Table 2, bioplanning was performed by pooling and phage amplification. More specifically, a total of four conditions (A-D) were set and performed through a combination of: a condition (pre-clearing) of clearing non-specific binding (Fc binder) by mixing phage with 50 µL of protein A and immunoglobulin complex and performing incubation at 37 °C for 1 hour; a condition of performing incubation at 37 °C for 4 hours using PBS (pH 7.4) or 50mM sodium acetate (pH 5.5) as a binding buffer; and a condition of performing competition elution with antigen in the step of elution, washing with 0.5% tween20 in PBS, then adding 100 µL of anti-CNTN4 chicken serum, and washing adding elution (Table 3). For each condition, 1 round to 3 round or up to 5 round were performed while controlling the stringency of the phage binding condition with respect to the antigen (20 µg to 1 µg of antigens).

**[Table 3]**

| | Phage display strategy | | | | |
|---|---|---|---|---|---|
| **Condition** | | **A** | **B** | **C** | **D** |
| Pre-clearing | | Yes | No | No | No |
| Binding buffer | | PBS (pH 7.4) | PBS (pH 7.4) | PBS (pH 7.4) | 50mM Sodium acetate (pH 5.5) |
| Elution | | Antigen & Serum | Antigen & Serum | Antigen | Antigen & Serum |

For selecting a candidate antibody, after the phage rescue is done in the output colony of 3 or 5 round, for example, a single colony was spotted and put into a medium, cultured to an OD600nm of 0.7 to 0.8, infected with helper phage, then cultured overnight, and centrifuged to obtain a phage culture medium, and then phage ELISA was performed. For the phage ELISA, first, a plate was coated overnight with antigens (mouse CNTN4 or human CNTN4) at 4 °C, followed by blocking with 3% BSA in PBS for 1 hour. Thereafter, 50µL of phage was added to the plate coated with antigens, followed by incubation for 2 hours. After the plate was incubated with anti-HA-HRP, which is a secondary antibody, for 1 hour, ABTS was added thereto and color-developed to measure absorbance at 405 nm. A total of 950 colonies from chicken number 1-4 and 3-2 libraries, and a total of 380 colonies from chicken number 1-3 and 3-5 libraries were subjected to ELISA screening, 138 and 306 colonies were respectively selected as a positive clone which binds to the antigen CNTN4, and 26 and 35 unique scFV sequences were selected through sequencing for identifying the scFv sequence.

### 1.2 Expression and Purification of scFv

For the functional evaluation of the scFv antibody selected in Example 1.1, cloning was performed in the form of scFv labeled with constant human kappa (hCk) in the form of fusion protein, or human IgG4 (S228P), and transient transfection was performed on Expi293F cells. The secreted scFv-hCk or IgG was bound to kappa select in the culture medium and then purified by pH elution, changed to PBS buffer through dialysis, and the protein was quantified to secure scFv-hCk or IgG.

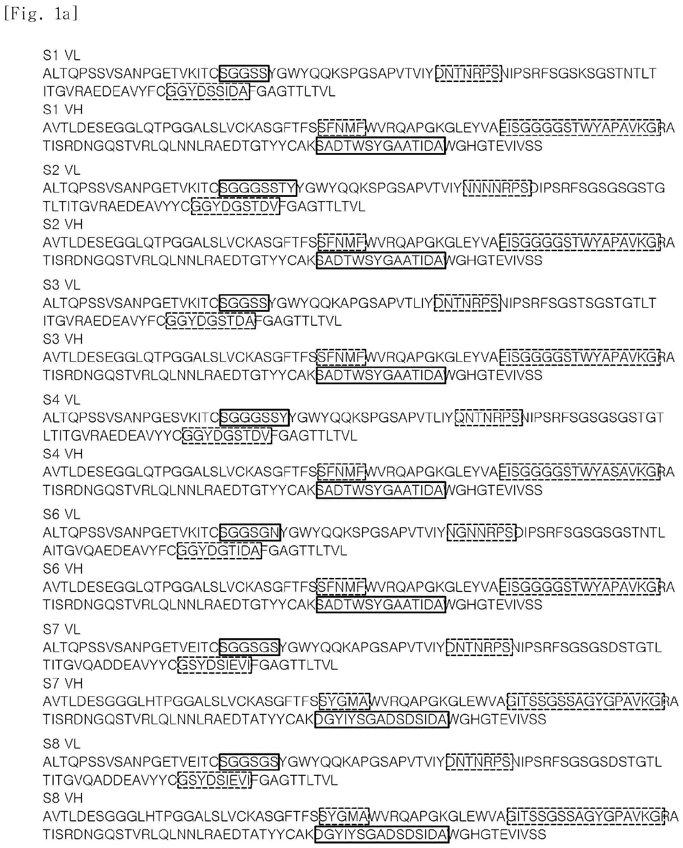

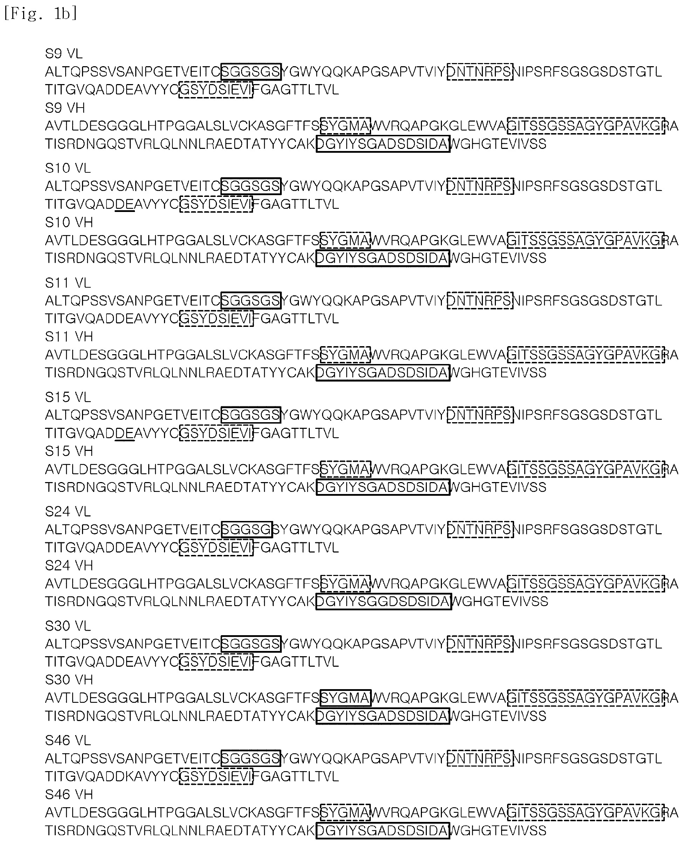

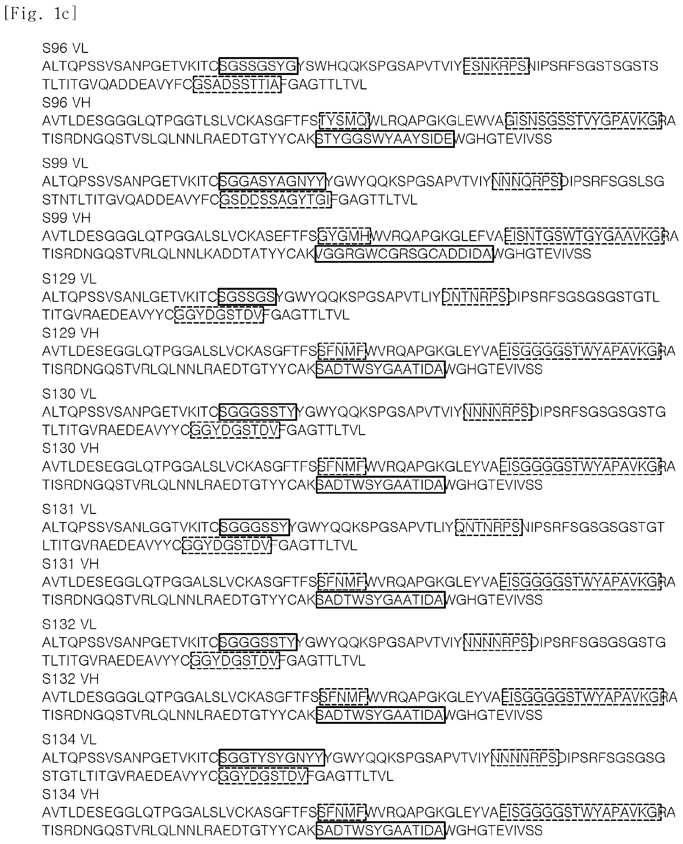

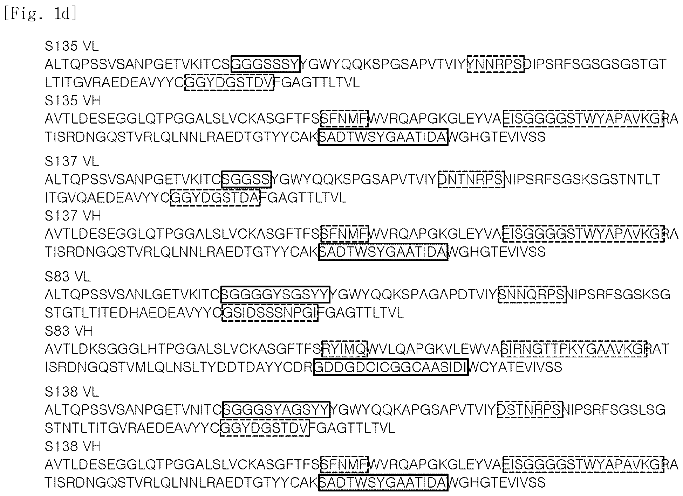

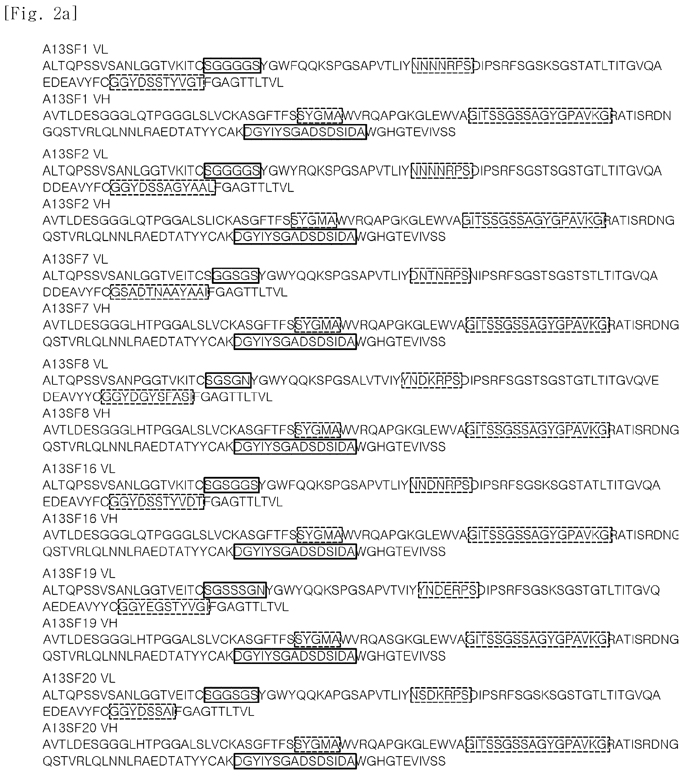

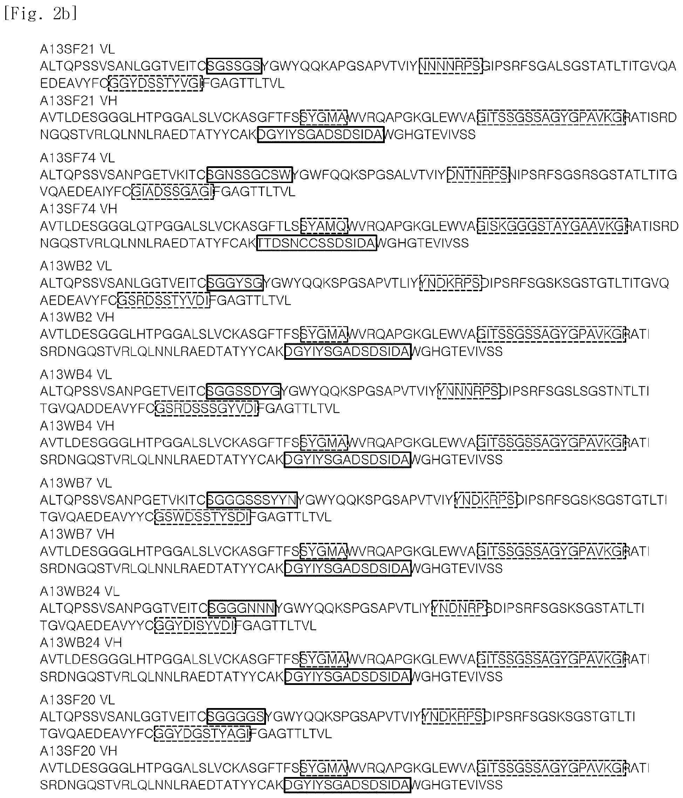

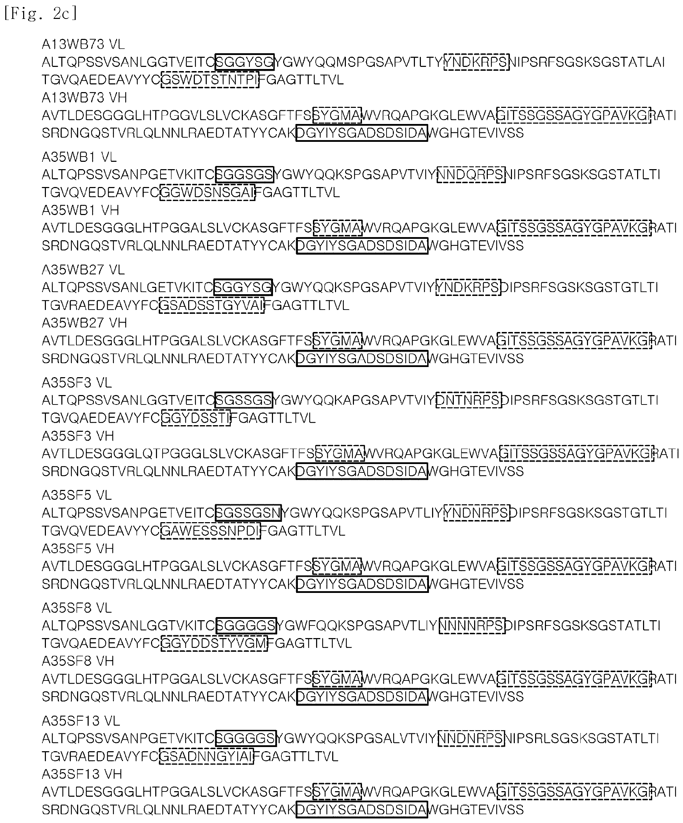

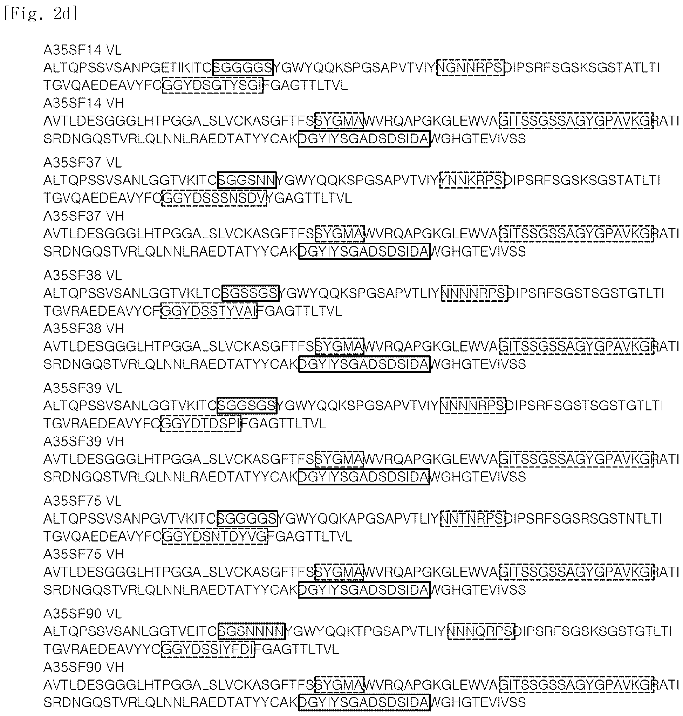

As a result, it was confirmed that a total of 25 scFv-hCks and a total of 27 IgG4s were expressed, and the amino acid sequences of the light chain and heavy chain variable regions of each of the expressed scFvs were shown in FIGS. 1 and 2, and Tables 4 and 5 (the sequences shown in FIGS. 1 and 2 that are indicated by solid and dashed lines represent CDR sequences of each region).

**[Table 4]**

| SEQ ID NO | Region | Sequence |
|---|---|---|
| SEQ ID NO: 1 | S1 light chain variable region (VL) | ALTQPSSVSANPGETVKITCSGGSSYGWYQQKSPGSAPVTVIYD NTNRPSNIPSRFSGSKSGSTNTLTITGVRAEDEAVYFCGGYDSSI DAFGAGTTLTVL |
| SEQ ID NO: 2 | S1 heavy chain variable region (VH) | AVTLDESEGGLQTPGGALSLVCKASGFTFSSFNMFWVRQAPGK GLEYVAEISGGGGSTWYAPAVKGRATISRDNGQSTVRLQLNNLR AEDTGTYYCAKSADTWSYGAATIDAWGHGTEVIVSS |
| SEQ ID NO: 3 | S2 light chain variable region (VL) | ALTQPSSVSANPGETVKITCSGGGSSTYYGWYQQKSPGSAPVT VIYNNNNRPSDIPSRFSGSGSGSTGTLTITGVRAEDEAVYYCGGY DGSTDVFGAGTTLTVL |
| SEQ ID NO: 2 | S2 heavy chain variable region (VH) | AVTLDESEGGLQTPGGALSLVCKASGFTFSSFNMFWVRQAPGK GLEYVAEISGGGGSTWYAPAVKGRATISRDNGQSTVRLQLNNLR AEDTGTYYCAKSADTWSYGAATIDAWGHGTEVIVSS |
| SEQ ID NO: 4 | S3 light chain variable region (VL) | ALTQPSSVSANPGETVKITCSGGSSYGWYQQKAPGSAPVTLIYD NTNRPSNIPSRFSGSTSGSTGTLTITGVRAEDEAVYFCGGYDGST DAFGAGTTLTVL |
| SEQ ID NO: 2 | S3 heavy chain variable region (VH) | AVTLDESEGGLQTPGGALSLVCKASGFTFSSFNMFWVRQAPGK GLEYVAEISGGGGSTWYAPAVKGRATISRDNGQSTVRLQLNNLR AEDTGTYYCAKSADTWSYGAATIDAWGHGTEVIVSS |
| SEQ ID NO: 5 | S4 light chain variable region (VL) | ALTQPSSVSANPGESVKITCSGGGSSYYGWYQQKSPGSAPVTLI YQNTNRPSNIPSRFSGSGSGSTGTLTITGVRAEDEAVYYCGGYD GSTDVFGAGTTLTVL |
| SEQ ID NO: 6 | S4 heavy chain variable region (VH) | AVTLDESEGGLQTPGGALSLVCKASGFTFSSFNMFWVRQAPGK GLEYVAEISGGGGSTWYASAVKGRATISRDNGQSTVRLQLNNL RAEDTGTYYCAKSADTWSYGAATIDAWGHGTEVIVSS |
| SEQ ID NO: 7 | S6 light chain variable region (VL) | ALTQPSSVSANPGETVKITCSGGSGNYGWYQQKSPGSAPVTVIY NGNNRPSDIPSRFSGSGSGSTNTLAITGVQAEDEAVYFCGGYDG TIDAFGAGTTLTVL |
| SEQ ID NO: 2 | S6 heavy chain variable region (VH) | AVTLDESEGGLQTPGGALSLVCKASGFTFSSFNMFWVRQAPGK GLEYVAEISGGGGSTWYAPAVKGRATISRDNGQSTVRLQLNNLR AEDTGTYYCAKSADTWSYGAATIDAWGHGTEVIVSS |
| SEQ ID NO: 8 | S7 light chain variable region (VL) | ALTQPSSVSANPGETVEITCSGGSGSYGWYQQKAPGSAPVTVIY DNTNRPSNIPSRFSGSGSDSTGTLTITGVQADDEAVYYCGSYDSI |
| | | EVIFGAGTTLTVL |
| SEQ ID NO: 9 | S7 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 8 | S8 light chain variable region (VL) | ALTQPSSVSANPGETVEITCSGGSGSYGWYQQKAPGSAPVTVIY DNTNRPSNIPSRFSGSGSDSTGTLTITGVQADDEAVYYCGSYDSI EVIFGAGTTLTVL |
| SEQ ID NO: 9 | S8 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 8 | S9 light chain variable region (VL) | ALTQPSSVSANPGETVEITCSGGSGSYGWYQQKAPGSAPVTVIY DNTNRPSNIPSRFSGSGSDSTGTLTITGVQADDEAVYYCGSYDSI EVIFGAGTTLTVL |
| SEQ ID NO: 9 | S9 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 8 | S10 light chain variable region (VL) | ALTQPSSVSANPGETVEITCSGGSGSYGWYQQKAPGSAPVTVIY DNTNRPSNIPSRFSGSGSDSTGTLTITGVQADDEAVYYCGSYDSI EVIFGAGTTLTVL |
| SEQ ID NO: 9 | S10 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 8 | S11 light chain variable region (VL) | ALTQPSSVSANPGETVEITCSGGSGSYGWYQQKAPGSAPVTVIY DNTNRPSNIPSRFSGSGSDSTGTLTITGVQADDEAVYYCGSYDSI EVIFGAGTTLTVL |
| SEQ ID NO: 9 | S11 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 8 | S15 light chain variable region (VL) | ALTQPSSVSANPGETVEITCSGGSGSYGWYQQKAPGSAPVTVIY DNTNRPSNIPSRFSGSGSDSTGTLTITGVQADDEAVYYCGSYDSI EVIFGAGTTLTVL |
| SEQ ID NO: 9 | S15 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 8 | S24 light chain variable region (VL) | ALTQPSSVSANPGETVEITCSGGSGSYGWYQQKAPGSAPVTVIY DNTNRPSNIPSRFSGSGSDSTGTLTITGVQADDEAVYYCGSYDSI EVIFGAGTTLTVL |
| SEQ ID NO: 10 | S24 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGGDSDSIDAWGHGTEVIVSS |
| SEQ ID NO: 8 | S30 light chain variable region (VL) | ALTQPSSVSANPGETVEITCSGGSGSYGWYQQKAPGSAPVTVIY DNTNRPSNIPSRFSGSGSDSTGTLTITGVQADDEAVYYCGSYDSI EVIFGAGTTLTVL |
| SEQ ID NO: 9 | S30 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 11 | S46 light chain variable region (VL) | ALTQPSSVSANPGETVEITCSGGSGSYGWYQQKAPGSAPVTVIY DNTNRPSNIPSRFSGSGSDSTGTLTITGVQADDKAVYYCGSYDSI |
| | | EVIFGAGTTLTVL |
| SEQ ID NO: 9 | S46 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 12 | S83 light chain variable region (VL) | ALTQPSSVSANLGETVKITCSGGGGYSGSYYYGWYQQKSPAGA PDTVIYSNNQRPSNIPSRFSGSKSGSTGTLTITEDHAEDEAVYYC GSIDSSSNPGIFGAGTTLTVL |
| SEQ ID NO: 13 | S83 heavy chain variable region (VH) | AVTLDKSGGGLHTPGGALSLVCKASGFTFSRYIMQWVLQAPGK VLEWVASIRNGTTPKYGAAVKGRATISRDNGQSTVMLQLNSLT YDDTDAYYCDRGDDGDCICGGCAASIDIWCYATEVIVSS |
| SEQ ID NO: 14 | S96 light chain variable region (VL) | ALTQPSSVSANPGETVKITCSGSSGSYGYSWHQQKSPGSAPVTV IYESNKRPSNIPSRFSGSTSGSTSTLTITGVQADDEAVYFCGSADS STTIAFGAGTTLTVL |
| SEQ ID NO: 15 | S96 heavy chain variable region (VH) | AVTLDESGGGLQTPGGTLSLVCKASGFTFSTYSMQWLRQAPGK GLEWVAGISNSGSSTVYGPAVKGRATISRDNGQSTVSLQLNNLR AEDTGTYYCAKSTYGGSWYAAYSIDEWGHGTEVIVSS |
| SEQ ID NO: 16 | S99 light chain variable region (VL) | ALTQPSSVSANPGETVKITCSGGASYAGNYYYGWYQQKSPGSA PVTVIYNNNQRPSDIPSRFSGSLSGSTNTLTITGVQADDEAVYFC GSDDSSAGYTGIFGAGTTLTVL |
| SEQ ID NO: 17 | S99 heavy chain variable region (VH) | AVTLDESGGGLQTPGGALSLVCKASEFTFSGYGMHWVRQAPG KGLEFVAEISNTGSWTGYGAAVKGRATISRDNGQSTVRLQLNN LKADDTATYYCAKVGGRGWCGRSGCADDIDAWGHGTEVIVSS |
| SEQ ID NO: 18 | S129 light chain variable region (VL) | ALTQPSSVSANLGETVKITCSGSSGSYGWYQQKSPGSAPVTLIY DNTNRPSDIPSRFSGSGSGSTGTLTITGVRAEDEAVYYCGGYDG STDVFGAGTTLTVL |
| SEQ ID NO: 2 | S129 heavy chain variable region (VH) | AVTLDESEGGLQTPGGALSLVCKASGFTFSSFNMFWVRQAPGK GLEYVAEISGGGGSTWYAPAVKGRATISRDNGQSTVRLQLNNLR AEDTGTYYCAKSADTWSYGAATIDAWGHGTEVIVSS |
| SEQ ID NO: 3 | S130 light chain variable region (VL) | ALTQPSSVSANPGETVKITCSGGGSSTYYGWYQQKSPGSAPVT VIYNNNNRPSDIPSRFSGSGSGSTGTLTITGVRAEDEAVYYCGGY DGSTDVFGAGTTLTVL |
| SEQ ID NO: 2 | S130 heavy chain variable region (VH) | AVTLDESEGGLQTPGGALSLVCKASGFTFSSFNMFWVRQAPGK GLEYVAEISGGGGSTWYAPAVKGRATISRDNGQSTVRLQLNNLR AEDTGTYYCAKSADTWSYGAATIDAWGHGTEVIVSS |
| SEQ ID NO: 19 | S131 light chain variable region (VL) | ALTQPSSVSANLGGTVKITCSGGGSSYYGWYQQKSPGSAPVTLI YQNTNRPSNIPSRFSGSGSGSTGTLTITGVRAEDEAVYYCGGYD GSTDVFGAGTTLTVL |
| SEQ ID NO: 2 | S131 heavy chain variable region (VH) | AVTLDESEGGLQTPGGALSLVCKASGFTFSSFNMFWVRQAPGK GLEYVAEISGGGGSTWYAPAVKGRATISRDNGQSTVRLQLNNLR AEDTGTYYCAKSADTWSYGAATIDAWGHGTEVIVSS |
| SEQ ID NO: 3 | S132 light chain variable region (VL) | ALTQPSSVSANPGETVKITCSGGGSSTYYGWYQQKSPGSAPVT VIYNNNNRPSDIPSRFSGSGSGSTGTLTITGVRAEDEAVYYCGGY DGSTDVFGAGTTLTVL |
| SEQ ID NO: 20 | S132 heavy chain variable region (VH) | AVTLDESGGGLQTPGGALSLVCKASGFTFSSFNMFWVRQAPGK GLEYVAEISGGGGSTWYAPAVKGRATISRDNGQSTVRLQLNNLR AEDTGTYYCAKSADTWSYGAATIDAWGHGTEVIVSS |
| SEQ ID NO: 21 | S134 light chain variable region (VL) | ALTQPSSVSANPGETVKITCSGGTYSYGNYYYGWYQQKSPGSA PVTVIYNNNNRPSDIPSRFSGSGSGSTGTLTITGVRAEDEAVYYC |
| | | GGYDGSTDVFGAGTTLTVL |
| SEQ ID NO: 2 | S134 heavy chain variable region (VH) | AVTLDESEGGLQTPGGALSLVCKASGFTFSSFNMFWVRQAPGK GLEYVAEISGGGGSTWYAPAVKGRATISRDNGQSTVRLQLNNLR AEDTGTYYCAKSADTWSYGAATIDAWGHGTEVIVSS |
| SEQ ID NO: 22 | S135 light chain variable region (VL) | ALTQPSSVSANPGETVKITCSGGGSSSYYGWYQQKSPGSAPVTV IYYNNRPSDIPSRFSGSGSGSTGTLTITGVRAEDEAVYYCGGYDG STDVFGAGTTLTVL |
| SEQ ID NO: 2 | S135 heavy chain variable region (VH) | AVTLDESEGGLQTPGGALSLVCKASGFTFSSFNMFWVRQAPGK GLEYVAEISGGGGSTWYAPAVKGRATISRDNGQSTVRLQLNNLR AEDTGTYYCAKSADTWSYGAATIDAWGHGTEVIVSS |
| SEQ ID NO: 23 | S137 light chain variable region (VL) | ALTQPSSVSANPGETVKITCSGGSSYGWYQQKSPGSAPVTVIYD NTNRPSNIPSRFSGSKSGSTNTLTITGVQAEDEAVYYCGGYDGS TDAFGAGTTLTVL |
| SEQ ID NO: 2 | S137 heavy chain variable region (VH) | AVTLDESEGGLQTPGGALSLVCKASGFTFSSFNMFWVRQAPGK GLEYVAEISGGGGSTWYAPAVKGRATISRDNGQSTVRLQLNNLR AEDTGTYYCAKSADTWSYGAATIDAWGHGTEVIVSS |
| SEQ ID NO: 24 | S138 light chain variable region (VL) | ALTQPSSVSANPGETVNITCSGGGSYAGSYYYGWYQQKAPGSA PVTVIYDSTNRPSNIPSRFSGSLSGSTNTLTITGVRAEDEAVYYC GGYDGSTDVFGAGTTLTVL |
| SEQ ID NO: 2 | S138 heavy chain variable region (VH) | AVTLDESEGGLQTPGGALSLVCKASGFTFSSFNMFWVRQAPGK GLEYVAEISGGGGSTWYAPAVKGRATISRDNGQSTVRLQLNNLR AEDTGTYYCAKSADTWSYGAATIDAWGHGTEVIVSS |

**[Table 5]**

| SEQ ID NO | Region | Sequence |
|---|---|---|
| SEQ ID NO: 25 | A13WB2 light chain variable region (VL) | ALTQPSSVSANLGGTVEITCSGGYSGYGWYQQKSPGSAPVTLIY YNDKRPSDIPSRFSGSKSGSTGTLTITGVQAEDEAVYFCGSRDSS TYVDIFGAGTTLTVL |
| SEQ ID NO: 9 | A13WB2 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 26 | A13WB4 light chain variable region (VL) | ALTQPSSVSANPGETVEITCSGGSSDYGYGWYQQKSPGSAPVT VIYYNNNRPSDIPSRFSGSLSGSTNTLTITGVQADDEAVYFCGSR DSSSGYVDIFGAGTTLTVL |
| SEQ ID NO: 9 | A13WB4 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 27 | A13WB7 light chain variable region (VL) | ALTQPSSVSANPGETVKITCSGGGSSSYYNYGWYQQKSPGSAP VTVIYYNDKRPSDIPSRFSGSKSGSTGTLTITGVQAEDEAVYYCG SWDSSTYSDIFGAGTTLTVL |
| SEQ ID NO: 9 | A13WB7 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 28 | A13WB24 light chain variable region (VL) | ALTQPSSVSANPGGTVEITCSGGGNNNYGWYQQKSPGSAPVTLI YYNDNRPSDIPSRFSGSKSGSTATLTITGVQAEDEAVYYCGGYDI SYVDIFGAGTTLTVL |
| SEQ ID NO: 9 | A13WB24 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR |
| | | AEDTATYYCAKDGYIYSGADSDSIDAWGHGTEVIVSS |
| SEQ ID NO: 29 | A13WB25 light chain variable region (VL) | ALTQPSSVSANLGGTVEITCSGGGGSYGWYQQKSPGSAPVTVIY YNDKRPSDIPSRFSGSKSGSTGTLTITGVQAEDEAVYFCGGYDG STYAGIFGAGTTLTVL |
| SEQ ID NO: 9 | A13WB25 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 30 | A13WB73 light chain variable region (VL) | ALTQPSSVSANLGGTVEITCSGGYSGYGWYQQMSPGSAPVTLT YYNDKRPSNIPSRFSGSKSGSTATLAITGVQAEDEAVYYCGSWD TSTNTPIFGAGTTLTVL |
| SEQ ID NO: 31 | A13WB73 heavy chain variable region (VH) | AVTLDESGGGLHTPGGVLSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 32 | A13SF1 light chain variable region (VL) | ALTQPSSVSANLGGTVKITCSGGGGSYGWFQQKSPGSAPVTLIY NNNNRPSDIPSRFSGSKSGSTATLTITGVQAEDEAVYFCGGYDSS TYVGTFGAGTTLTVL |
| SEQ ID NO: 33 | A13SF1 heavy chain variable region (VH) | AVTLDESGGGLQTPGGGLSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 34 | A13SF2 light chain variable region (VL) | ALTQPSSVSANLGGTVKITCSGGGGSYGWYRQKSPGSAPVTLIY NNNNRPSDIPSRFSGSTSGSTGTLTITGVQADDEAVYFCGGYDSS AGYAALFGAGTTLTVL |
| SEQ ID NO: 35 | A13SF2 heavy chain variable region (VH) | AVTLDESGGGLQTPGGALSLICKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 36 | A13SF7 light chain variable region (VL) | ALTQPSSVSANLGGTVEITCSGGSGSYGWYQQKSPGSAPVTLIY DNTNRPSNIPSRFSGSTSGSTSTLTITGVQADDEAVYFCGSADTN AAYAAIFGAGTTLTVL |
| SEQ ID NO: 9 | A13SF7 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 37 | A13SF8 light chain variable region (VL) | ALTQPSSVSANPGGTVKITCSGSGNYGWYQQKSPGSALVTVIYY NDKRPSDIPSRFSGSTSGSTGTLTITGVQVEDEAVYYCGGYDGY SFASIFGAGTTLTVL |
| SEQ ID NO: 9 | A13SF8 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 38 | A13SF16 light chain variable region (VL) | ALTQPSSVSANLGGTVKITCSGSGGSYGWFQQKSPGSAPVTLIY NNDNRPSDIPSRFSGSKSGSTATLTITGVQAEDEAVYFCGGYDSS TYVDTFGAGTTLTVL |
| SEQ ID NO: 33 | A13SF16 heavy chain variable region (VH) | AVTLDESGGGLQTPGGGLSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 39 | A13SF19 light chain variable region (VL) | ALTQPSSVSANLGGTVEITCSGSSSGNYGWYQQKSPGSAPVTVI YYNDERPSDIPSRFSGSKSGSTGTLTITGVQAEDEAVYYCGGYE GSTYVGIFGAGTTLTVL |
| SEQ ID NO: 40 | A13SF19 heavy chain variable region (VH) | AVTLDESGGGLHTPGGALSLVCKASGFTFSSYGMAWVRQASGK GLEWVAGITSSGSSAGYGPAVKGRATISRDNGQSTVRLQLNNLR |
| | | AEDTATYYCAKDGYIYSGADSDSIDAWGHGTEVIVSS |
| SEQ ID NO: 41 | A13SF20 light chain variable region (VL) | ALTQPSSVSANLGGTVEITCSGGSGSYGWYQQKAPGSAPVTLIY NSDKRPSDIPSRFSGSKSGSTGTLTITGVQAEDEAVYFCGGYDSS AIFGAGTTLTVL |
| SEQ ID NO: 9 | A13SF20 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 42 | A13SF21 light chain variable region (VL) | ALTQPSSVSANLGGTVEITCSGSSGSYGWYQQKAPGSAPVTVIY NNNNRPSGIPSRFSGALSGSTATLTITGVQAEDEAVYFCGGYDSS TYVGIFGAGTTLTVL |
| SEQ ID NO: 9 | A13SF21 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 43 | A13SF74 light chain variable region (VL) | ALTQPSSVSANPGETVKITCSGNSSGCSWYGWFQQKSPGSALVT VIYDNTNRPSNIPSRFSGSRSGSTATLTITGVQAEDEAIYFCGIAD SSGAGIFGAGTTLTVL |
| SEQ ID NO: 44 | A13SF74 heavy chain variable region (VH) | AVTLDESGGGLQTPGGALSLVCKASGFTLSSYAMQWVRQAPG KGLEWVAGISKGGGSTAYGAAVKGRATISRDNGQSTVRLQLNN LRAEDTATYFCAKTTDSNCCSSDSIDAWGHGTEVIVSS |
| SEQ ID NO: 45 | A3 5WB 1 light chain variable region (VL) | ALTQPSSVSANPGETVKITCSGGSGSYGWYQQKSPGSAPVTVIY NNDQRPSNIPSRFSGSKSGSTATLTITGVQVEDEAVYFCGGWDS NSGAIFGAGTTLTVL |
| SEQ ID NO: 9 | A35WB1 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 46 | A35WB27 light chain variable region (VL) | ALTQPSSVSANLGETVKITCSGGYSGYGWYQQKSPGSAPVTVIY YNDKRPSDIPSRFSGSKSGSTGTLTITGVRAEDEAVYFCGSADSS TGYVAIFGAGTTLTVL |
| SEQ ID NO: 9 | A35WB27 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 47 | A35SF3 light chain variable region (VL) | ALTQPSSVSANLGGTVEITCSGSSGSYGWYQQKAPGSAPVTVIY DNTNRPSDIPSRFSGSKSGSTGTLTITGVQAEDEAVYFCGGYDSS TIFGAGTTLTVL |
| SEQ ID NO: 33 | A35SF3 heavy chain variable region (VH) | AVTLDESGGGLQTPGGGLSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 48 | A35SF5 light chain variable region (VL) | ALTQPSSVSANPGETVEITCSGSSGSNYGWYQQKSPGSAPVTLI YYNDNRPSDIPSRFSGSKSGSTGTLTITGVQVEDEAVYYCGAWE SSSNPDIFGAGTTLTVL |
| SEQ ID NO: 9 | A35SF5 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 49 | A35SF8 light chain variable region (VL) | ALTQPSSVSANLGGTVKITCSGGGGSYGWFQQKSPGSAPVTLIY NNNNRPSDIPSRFSGSKSGSTATLTITGVQAEDEAVYFCGGYDDS TYVGMFGAGTTLTVL |
| SEQ ID NO: 9 | A35SF8 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR |
| | | AEDTATYYCAKDGYIYSGADSDSIDAWGHGTEVIVSS |
| SEQ ID NO: 50 | A35SF13 light chain variable region (VL) | ALTQPSSVSANPGETVKITCSGGGGSYGWYQQKSPGSALVTVIY NNDNRPSNIPSRLSGSKSGSTATLTITGVRAEDEAVYFCGSADNN GYIAIFGAGTTLTVL |
| SEQ ID NO: 9 | A35SF13 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 51 | A35SF14 light chain variable region (VL) | ALTQPSSVSANPGETIKITCSGGGSYGWYQQKSPGSAPVTVIY NGNNRPSDIPSRFSGSKSGSTATLTITGVQAEDEAVYFCGGYDSG TYSGIFGAGTTLTVL |
| SEQ ID NO: 9 | A35SF14 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 52 | A35SF37 light chain variable region (VL) | ALTQPSSVSANLGGTVKITCSGGSNNYGWYQQKSPGSAPVTVI YYNNKRPSDIPSRFSGSKSGSTATLTITGVQAEDEAVYFCGGYDS SSNSDVYGAGTTLTVL |
| SEQ ID NO: 9 | A35SF37 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 53 | A35SF38 light chain variable region (VL) | ALTQPSSVSANLGGTVKLTCSGSSGSYGWYQQKSPGSAPVTLIY NNNNRPSDIPSRFSGSTSGSTGTLTITGVRAEDEAVYFCGGYDSS TYVAIFGAGTTLTVL |
| SEQ ID NO: 9 | A35SF38 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 54 | A35SF39 light chain variable region (VL) | ALTQPSSVSANLGGTVKITCSGGSGSYGWYQQKSPGSAPVTVIY NNNNRPSDIPSRFSGSTSGSTGTLTITGVRAEDEAVYFCGGYDTD SPIFGAGTTLTVL |
| SEQ ID NO: 9 | A35SF39 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 55 | A35SF75 light chain variable region (VL) | ALTQPSSVSANPGVTVKITCSGGGGSYGWYQQKAPGSAPVTLI YNNTNRPSDIPSRFSGSRSGSTNTLTITGVQAEDEAVYFCGGYDS NTDYVGFGAGTTLTVL |
| SEQ ID NO: 9 | A35SF75 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |
| SEQ ID NO: 56 | A35SF90 light chain variable region (VL) | ALTQPSSVSANLGGTVEITCSGSNNNNYGWYQQKTPGSAPVTLI YNNNQRPSDIPSRFSGSKSGSTGTLTITGVRAEDEAVYYCGGYD SSIYFDIFGAGTTLTVL |
| SEQ ID NO: 9 | A35SF90 heavy chain variable region (VH) | AVTLDESGGGLHTPGGALSLVCKASGFTFSSYGMAWVRQAPGK GLEWVAGITSSGSSAGYGPAVKGRATISRDNGQSTVRLQLNNLR AEDTATYYCAKDGYIYSGADSDSIDAWGHGTEVIVSS |

### 1.3 Confirmation of CNTN4 Binding Ability of Selected scFv Antibodies

### (1) Confirmation of Binding Ability of scFv to Antigenic Protein according to ELISA

An ELISA tests were performed on a total of 25 scFv-hCks and a total of 27 IgGs for which the expression was confirmed in Example 1.2. An ELISA plate was coated with 200 ng of antigens (human CNTN4-rabbit Fc or mouse CNTN4-rabbit Fc). 200 ng of a negative control (human antigen-rabbit Fc) for a tagging protein was used. For antibody binding, anti-CNTN4 scFv-hCk was serially diluted from a maximum of 1 µM to 0.001 nM by 1/10× each, and anti-CNTN4 IgG was serially diluted from a maximum of 10 nM to 0.00001 nM by 1/10× each, thereby binding to the antigen. Anti-human kappa-HRP or anti-human IgG Fc-HRP was used as the secondary antibody for antibody detection. Color was developed using ABTS, which is a color developing reagent, and the absorbance at 405 nm was measured.

As shown in Tables 6 and 7, the results showed that reactivity to most antigens was good, and there existed many clones showing similar reactivity to both human and mouse antigens.

**[Table 6]**

| EC₅₀, nM | Negative control | Human CNTN4 | Mouse CNTN4 |
|---|---|---|---|
| S1 | N/A | 4.44 | 1.53 |
| S2 | N/A | 0.12 | 0.10 |
| S4 | N/A | 2.60 | 1.15 |
| S6 | N/A | 0.37 | 1.85 |
| S7 | N/A | 1.51 | 1.20 |
| S8 | N/A | 1.10 | 1.53 |
| S9 | N/A | 0.08 | 0.09 |
| S10 | N/A | 1.82 | 7.00 |
| S11 | N/A | 1.44 | 2.39 |
| S15 | N/A | 1.35 | 2.12 |
| S24 | N/A | 1.12 | 2.07 |
| S30 | N/A | 1.15 | 1.12 |
| S46 | N/A | 1.22 | 1.20 |
| S99 | N/A | 8.34 | 3.64 |
| S129 | N/A | 0.55 | 0.37 |
| S130 | N/A | 0.81 | 0.46 |
| S131 | N/A | 0.74 | 0.56 |
| S132 | N/A | 1.16 | 0.72 |
| S134 | N/A | 0.75 | 0.75 |
| S135 | N/A | 1.06 | 0.71 |
| S137 | N/A | 0.41 | 0.52 |
| S138 | N/A | 2.37 | 1.06 |

**[Table 7]**

| EC₅₀, nM | Negative control | Human CNTN4 | Mouse CNTN4 |
|---|---|---|---|
| A13WB2 | N/A | 0.01 | 0.01 |
| A13WB4 | N/A | 0.01 | 0.01 |
| A13WB7 | N/A | 0.01 | 0.01 |
| A13WB24 | N/A | 0.01 | 0.01 |
| A13WB25 | N/A | 0.01 | 0.01 |
| A13WB73 | N/A | 0.01 | 0.01 |
| A13SF1 | N/A | 0.01 | 0.02 |
| A13SF2 | N/A | 0.01 | 0.01 |
| A13SF7 | N/A | 0.01 | 0.01 |
| A13SF8 | N/A | 0.01 | 0.01 |
| A13SF16 | N/A | 0.01 | 0.01 |
| A13SF19 | N/A | 0.01 | 0.01 |
| A13SF20 | N/A | 0.01 | 0.01 |
| A13SF21 | N/A | 0.01 | 0.01 |
| A13SF74 | N/A | 0.11 | 0.12 |
| A35WB1 | N/A | 0.02 | 0.01 |
| A35WB27 | N/A | 0.01 | 0.01 |
| A35SF3 | N/A | 0.01 | 0.01 |
| A35SF5 | N/A | 0.01 | 0.01 |
| A35SF8 | N/A | 0.01 | 0.02 |
| A35SF13 | N/A | 0.01 | 0.02 |
| A35SF14 | N/A | 0.01 | 0.01 |
| A35SF37 | N/A | 0.01 | 0.01 |
| A35SF38 | N/A | 0.01 | 0.01 |
| A35SF39 | N/A | 0.01 | 0.01 |
| A35SF75 | N/A | 0.21 | 0.20 |
| A35SF90 | N/A | 0.01 | 0.01 |

### (3) Confirmation of Binding Ability of scFv to Antigens Expressed on Cell Surfaces according to FACS

In 293FT cell, human CNTN4 antigen was expressed on the cell membrane via transient transfection and anti-CNTN4 scFv-hCk or anti-CNTN4 IgG was bound thereto at a concentration of 40 µg/mL. Dot shift was confirmed by the detection of anti-CNTN4 bound to the antigen with anti-hCk-PE or anti-hFc-PE by using FACS.

As shown in Tables 8 and 9, the results show that 2, 4, 99, 129, 130, 131, 132, 134, 135, 137, and 138 of the hCk-type scFv clones showing binding ability to human CNTN4 belong to the upper group (> 15.0%) with a large amount of dot shift, and all of the IgG-type anti-CNTN4 exhibit excellent binding ability of 75% or more.

**[Table 8]**

| Shift % | Un-transfected cell | Human CNTN4 transfected cell |
|---|---|---|
| Secondary antibody only | 1.11 | 0.48 |
| S1 | 0.21 | 13.8 |
| S2 | 0.26 | 15.1 |
| S3 | 0.18 | 13.9 |
| S4 | 0.20 | 15.4 |
| S6 | 0.22 | 14.7 |
| S7 | 0.20 | 6.42 |
| S8 | 0.21 | 12.9 |
| S9 | 0.22 | 4.09 |
| S10 | 0.21 | 6.95 |
| S11 | 0.20 | 5.29 |
| S15 | 0.23 | 6.66 |
| S24 | 0.28 | 5.63 |
| S30 | 0.24 | 6.87 |
| S46 | 0.19 | 8.19 |
| S83 | 0.13 | 5.27 |
| S96 | 0.25 | 1.69 |
| S99 | 3.75 | 20.5 |
| S129 | 0.20 | 16.0 |
| S130 | 0.19 | 16.4 |
| S131 | 0.24 | 16.6 |
| S132 | 0.18 | 16.1 |
| S134 | 0.15 | 18.0 |
| S135 | 0.14 | 18.2 |
| S137 | 0.19 | 15.3 |
| S138 | 0.18 | 15.8 |

**[Table 9]**

| Shift % | Un-transfected cell | Human CNTN4 transfected cell |
|---|---|---|
| Second antibody only | 0.81 | 1.25 |
| A13WB2 | 7.74 | 86.6 |
| A13WB4 | 1.13 | 84.7 |
| A13WB7 | 5.47 | 86.4 |
| A13WB24 | 1.6 | 85.1 |
| A13WB25 | 0.65 | 82.4 |
| A13WB73 | 0.72 | 86.4 |
| A13SF1 | 0.52 | 83.8 |
| A13SF2 | 4.24 | 83.5 |
| A13SF7 | 0.42 | 83.2 |
| A13SF8 | 17.6 | 90.4 |
| A13SF16 | 6.23 | 84.8 |
| A13SF19 | 0.77 | 79.6 |
| A13SF20 | 0.42 | 82.2 |
| A13SF21 | 0.36 | 80.4 |
| A13SF74 | 1.11 | 84.1 |
| A35WB1 | 3.93 | 83.9 |
| A35WB27 | 9.39 | 88.2 |
| A35SF3 | 0.75 | 81.9 |
| A35SF5 | 0.39 | 82.6 |
| A35SF8 | 0.55 | 80.4 |
| A35SF13 | 1.59 | 82.1 |
| A35SF14 | 0.58 | 81.6 |
| A35SF37 | 5.96 | 86.6 |
| A35SF38 | 0.72 | 77.9 |
| A35SF39 | 3.87 | 80 |
| A35SF75 | 0.39 | 84.8 |
| A35SF90 | 0.49 | 82.8 |

### Example 2: Preparation of Anti-CNTN4 Chicken-human Chimeric IgG4 Antibodies and Confirmation of Binding Ability thereof

### 2.1 Preparation of Anti-CNTN4 Chicken-human Chimeric IgG4-S228P, A-01

For the production of antibodies in the form of human chimeric IgG, S129, of which binding ability was confirmed through Example 1, was selected and the light chain and heavy chain regions thereof were cloned to a bicistronic expression vector. DNA was transient transfected into Expi293F cells and cultured up to 50% of viability to perform IgG expression, and the culture medium was bound to Kappa select resin (kappa region capture) to elute and primarily purify IgG. Thereafter, anti-CNTN4 129 chimeric IgG4-S228P (hereinafter referred to as "A-01") having a purity of 72.4% on SEC-HPLC was prepared by binding to Mabselect (Fc region capture) and eluting and secondarily purifying IgG in order to clear light chain impurities.

### 2.2 Confirmation of Binding Ability of A-01 to Antigenic Protein according to ELISA

An ELISA test was performed on A-01 prepared in Example 2.1. An ELISA plate was coated with 200 ng of antigens (human CNTN4-10xHis or mouse CNTN4-10xHis). For antibody binding, A-01 was serially diluted from a maximum of 1 µM to 0.0001 nM, thereby binding to the antigen. Anti-human Fc-HRP was used as a secondary antibody for antibody detection. Color was developed using ABTS, which is a color developing reagent, and the absorbance at 405 nm was measured. The resultant binding abilities are shown in Table 10.

**[Table 10]**

| EC₅₀, nM | Human CNTN4 | Mouse CNTN4 |
|---|---|---|
| A-01 | 0.011 | 0.011 |

### 2.3 Confirmation of Binding Ability of A-01 to Antigenic Protein according to Surface Plasmon Resonance (SPR)

An SPR test was performed on A-01 prepared in Example 2.1.

Specifically, in order to bind anti-human IgG Fc (capture antibody) using amine coupling, CM5 chip was mounted, running buffer was allowed to flow, the chip was activated, and then capture antibody was attached thereto. Dextrin to which capture antibody was not attached was deactivated using ethanolamine, and then regeneration was performed using 3M magnesium chloride to clear analyte except capture antibody. The A-01 prepared at a concentration of 0.05 µg/mL was allowed to flow for 30 seconds at a rate of 10 µL/min for binding. Thereafter, human CNTN4 (400 nM to 0.78 nM) prepared by sequential dilution as an antigen was allowed to flow at a rate of 30 µL/min for 3 minutes to be bound, and dissociation was performed for 5 minutes. Then, in order to clear all analytes attached to the capture antibody, a regeneration solution (3M magnesium chloride) was allowed to flow at a rate of 20 µL/min for 30 seconds, and regeneration was performed. For each concentration of antigen, binding of A-01, binding of antigen, dissociation, and regeneration were repeated in order. The results are shown in Table 11.

**[Table 11]**

| **Antigen** | **Dissociation constant Kd** |
|---|---|
| Human CNTN-4 protein | 3.365 X 10⁻⁸ M |

From this, it was confirmed that the antibody of the present invention binds to the human CNTN4 protein with high affinity.

### Example 3: Preparation of Anti-CNTN4 Humanized Antibodies and Confirmation of Binding Ability

### 3.1 Preparation of Anti-CNTN4 Humanized Antibodies

Humanized antibodies were prepared using A-01.

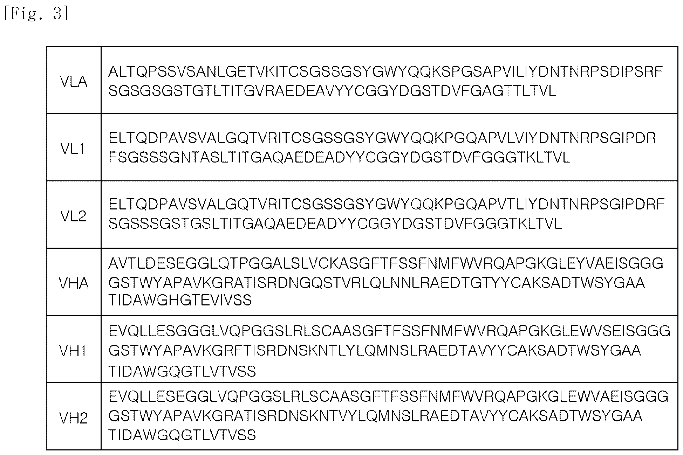

Specifically, in the variable region original sequences (VLA and VHA) of A-01, sequences (VL1 and VH1) that fully changed the framework region to the human framework region were combined with sequences (VL2 and VH2) in which some A-01 original sequences were left in VL1 and VH1 (Table 12 and FIG. 3). When comparing A-01 with fully humanized antibodies (VL1 and VH1), it showed about 78% humanization. When comparing fully humanized antibodies (VL1 and VH1) with antibodies of VL2 and VH2 combination having some back mutation sequences, at least 96% of the sequences were identical. When DNA was transient transfected into Expi293F cells and cultured in order to prepare a total of eight humanized antibodies (LAH1, LAH2, L1HA, L1H1, L1H2, L2HA, L2H1, and L2H2), except A-01 (LAHA), in the form of IgG4, antibodies (LAH1, L1H1, and L2H1) having VH1 in which the heavy chain variable region was fully humanized were not expressed. LAH2, L1H2, and L2H2, which are the antibodies having VH2, were then purified to secure 4 mg, 11 mg, and 12 mg, respectively.

**[Table 12]**

| SEQ ID NO | Region | Sequence |
|---|---|---|
| SEQ ID NO: 18 | VLA | ALTQPSSVSANLGETVKITCSGSSGSYGWYQQKSPGSAPVTLIYD NTNRPSDIPSRFSGSGSGSTGTLTITGVRAEDEAVYYCGGYDGSTD VFGAGTTLTVL |
| SEQ ID NO: 2 | VHA | AVTLDESEGGLQTPGGALSLVCKASGFTFSSFNMFWVRQAPGKG LEYVAEISGGGGSTWYAPAVKGRATISRDNGQSTVRLQLNNLRAE DTGTYYCAKSADTWSYGAATIDAWGHGTEVIVSS |
| SEQ ID NO: 156 | VL1 | ELTQDPAVSVALGQTVRITCSGSSGSYGWYQQKPGQAPVLVIYDN TNRPSGIPDRFSGSSSGNTASLTITGAQAEDEADYYCGGYDGSTD VFGGGTKLTVL |
| SEQ ID NO: 157 | VH1 | EVQLLESGGGLVQPGGSLRLSCAASGFTFSSFNMFWVRQAPGKG LEWVSEISGGGGSTWYAPAVKGRFTISRDNSKNTLYLQMNSLRAE DTAVYYCAKSADTWSYGAATIDAWGQGTLVTVSS |
| SEQ ID NO: 158 | VL2 | ELTQDPAVSVALGQTVRITCSGSSGSYGWYQQKPGQAPVTLIYDN TNRPSGIPDRFSGSSSGSTGSLTITGAQAEDEADYYCGGYDGSTD VFGGGTKLTVL |
| SEQ ID NO: 159 | VH2 | EVQLLESEGGLVQPGGSLRLSCAASGFTFSSFNMFWVRQAPGKG LEWVAEISGGGGSTWYAPAVKGRATISRDNSKNTVYLQMNSLRA EDTAVYYCAKSADTWSYGAATIDAWGQGTLVTVSS |

### 3.2 Confirmation of Binding Ability of Humanized Antibodies to Antigenic Proteins according to ELISA

The LAH2, L1H2, and L2H2 antibodies prepared in Example 3.1 were subjected to an ELISA test together with A-01. An ELISA plate was coated with 200 ng of antigens (human CNTN4-10xHis or mouse CNTN4-10xHis). For antibody binding, antibodies were serially diluted from a maximum of 1 µM to 0.0001 nM, thereby binding to the antigen. Anti-human Fc-HRP was used as a secondary antibody for antibody detection. Color was developed using ABTS, which is a color developing reagent, and the absorbance at 405 nm was measured.

The resultant binding abilities are shown in Table 13. It was confirmed that the most humanized L1H2 has a difference in binding ability with the CNTN4 protein within 3 times compared to A-01 origin.

**[Table 13]**

| EC₅₀, nM | Human CNTN4 | Mouse CNTN4 |
|---|---|---|
| A-01 | 0.011 | 0.011 |
| LAH2 | 0.017 | 0.017 |
| L1H2 | 0.028 | 0.029 |
| L2H2 | 0.018 | 0.019 |

### 3.3 Confirmation of Binding Ability of Humanized Antibodies to Antigenic Protein according to Surface Plasmon Resonance (SPR)

An SPR test was performed on L1H2, which is most humanized at about 98%, among the antibodies prepared in Example 3.1. More specifically, in order to bind anti-human IgG Fc (capture antibody) using amine coupling, CM5 chip was mounted, running buffer was allowed to flow, the chip was activated, and then capture antibody was attached thereto. Dextrin to which capture antibody was not attached was deactivated using ethanolamine, and then regeneration was performed using 3M magnesium chloride to clear analyte except capture antibody. The L1H2 prepared at a concentration of 0.05 µg/mL was allowed to flow for 30 seconds at a rate of 10 µL/min for binding. Thereafter, human CNTN4 (400 nM to 0.78 nM) prepared by sequential dilution as an antigen was allowed to flow at a rate of 30 µL/min for 3 minutes to be bound, and dissociation was performed for 5 minutes. Then, in order to clear all analytes attached to the capture antibody, a regeneration solution (3M magnesium chloride) was allowed to flow at a rate of 20 µL/min for 30 seconds, and regeneration was performed. For each concentration of antigen, binding of L1H2, binding of antigen, dissociation, and regeneration were repeated in order.

The results are shown in Table 14.

**[Table 14]**

| Antigen | Dissociation constant Kd |
|---|---|
| Human CNTN-4 protein | 9.319 X 10⁻⁸ M |

From this, it may be seen that even when A-01 antibody is 98% humanized, it has a sufficiently high binding ability to the CNTN4 protein.

### Example 4: Test about CNTN4 Neutralization Ability of A-01 and L1H2 Antibodies Using Human T Cells

A final 50 mL of a-CD3 antibody at a concentration of 4 mg/mL and a CNTN4 recombinant protein at a concentration of 150 nM were prepared and placed in a 96-well plate and incubated at 37 °C for about 16 hours.

50 mL of blood (contained in a blood collection tube treated with sodium heparin) per donor and PBS were mixed at a ratio of 1:1, 15 mL of Ficoll was previously contained in a 50 mL tube, and 30 mL of diluted blood was carefully placed thereon and stacked such that the Ficoll layer and the blood layer were separated. Centrifugation (acceleration and deceleration speeds set to 0) was performed for 25 minutes at 1,800 rpm and 20 °C, and the supernatant was removed such that a white buffy layer did not rise. The buffy layer was transferred to a new 50 mL tube, and the tube was filled to 50 mL with PBS, and centrifuged for 5 minutes at 1,800 rpm and 4 °C. 10 mL of 1 X RBC lysis buffer was added, dissolved well, and left at room temperature for 5 minutes. After filling up to 50 mL with PBS, centrifugation was performed for 5 minutes at 1,800 rpm and 4 °C, the supernatant was removed, and the pellets were dissolved with PBS, and cells (PBMC) were then observed through a microscope and the number of cells was calculated.

1,000 µL of MACS buffer per 2 × 10⁸ PBMC cells obtained in the above experiment was added, followed by resuspension. The solution was divided into two halves, one half of which was put into a CD4 tube, and one half of which was put into a CD8 tube. 50 µL of antibody cocktail per 1 × 10⁸ PBMCs of each group was resuspended and stored at room temperature for 10 minutes. 100 µL of anti-biotin microbeads per 1 × 10⁸ PBMCs was resuspended and stored at room temperature for 10 minutes. Meanwhile, MACS magnetic field was disinfected and placed in a clean bench, and two LS columns (for CD4 and CD8) were installed. After flowing 3 mL of MACS buffer into each column, the buffer passing through each column was discarded. The cells of each group after the reaction was completed were loaded onto two LS columns mounted on the magnetic field, respectively. When there was no more cell solution falling down, 3 mL of MACS buffer was allowed to flow into each column. Thereafter, cells in a solution that fell from each column were observed through a microscope and the number of cells was calculated.

18 µL of DMSO was added to 50 µg of CFSE and pipetted to make a concentration of 5 mM and use the cells. 1 mL of MTM media was added to the CD4 cells and CD8 cells obtained in the above experiment, CFSE was added to reach a final concentration of 5 µM, suspended, and then stored in a water bath at 37 °C for 10 minutes. The solution was centrifuged at 1,800 rpm and 4 °C for 5 minutes, and the supernatant was removed. Then, 1 mL of MTM media was added thereto and resuspended, and then centrifuged again at 1,800 rpm and 4 °C for 5 minutes, and the supernatant was removed. Finally, the cells were resuspended with MTM media to reach 2 × 10⁵ cells/200 µL per well to be used in the experiment.

Then, the incubated plate was washed twice with 200 µL of PBS, and 1.5 mM and 3 mM of A-01 and L1H2 were finally made 50 mL into CNTN4 treated wells, respectively, and placed into a 96-well plate. Incubation was performed at 37 °C for about 4 hours.

Thereafter, the incubated plate was washed twice with 200 mL of PBS, 200 mL of T cells per each well were added, and then stored in a 5% CO₂ incubator at 37 °C for 3 days. After 3 days, T cells were obtained and transferred to a FACS tube, and the degree of cells differentiated, which are stained with CFSE, was observed using a FACS instrument.

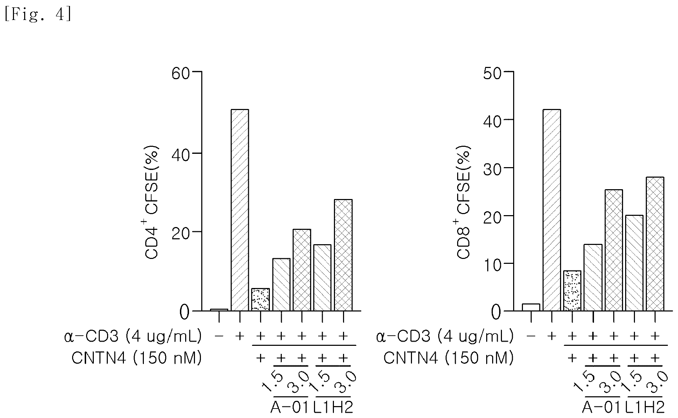

The results are shown in FIG. 4.

As shown in FIG. 4, it was confirmed that the CNTN4 recombinant protein inhibited the proliferation of CD4⁺ T cells and CD8⁺ T cells. It was observed that the proliferation of T cells, which had been inhibited by CNTN4, increased with the concentrations of treated A-01 and L1H2. This shows that A-01 and L1H2 of the present invention exhibit significantly excellent ability to neutralize CNTN4.

From this, it was confirmed that the CNTN4 antibodies of the present invention effectively neutralized CNTN4 and inhibited the T cell inhibitory function thereof.

## Claims

1. An anti-CNTN4 antibody or an antigen-binding fragment thereof comprising:
a light chain CDR1 comprising an amino acid sequence of SEQ ID NO: 70;
a light chain CDR2 comprising an amino acid sequence of SEQ ID NO: 88;
a light chain CDR3 comprising an amino acid sequence of SEQ ID NO: 116;
a heavy chain CDR1 comprising an amino acid sequence of SEQ ID NO: 58;
a heavy chain CDR2 comprising an amino acid sequence of SEQ ID NO: 89; and
a heavy chain CDR3 comprising an amino acid sequence of SEQ ID NO: 115.

2. An anti-CNTN4 antibody or an antigen-binding fragment thereof comprising:
a light chain variable region comprising an amino acid sequence of SEQ ID NO: 18; and
a heavy chain variable region comprising an amino acid sequence of SEQ ID NO: 2.

3. An anti-CNTN4 antibody or an antigen-binding fragment thereof comprising:
a light chain variable region comprising an amino acid sequence of SEQ ID NO: 156; and
a heavy chain variable region comprising an amino acid sequence of SEQ ID NO: 159.

4. The anti-CNTN4 antibody or antigen-binding fragment thereof of any one of claims 1 to 3, specifically binding to a human or mouse CNTN4 protein.

5. The anti-CNTN4 antibody or antigen-binding fragment thereof of any one of claims 1 to 3, binding to a human CNTN4 protein with a dissociation constant (Kd) of 1 × 10⁻⁷ M or less.

6. The anti-CNTN4 antibody or antigen-binding fragment thereof of any one of claims 1 to 3, increasing T cell activity.

7. The anti-CNTN4 antibody or antigen-binding fragment thereof of claim 6, wherein the T cells are CD4+ T cells or CD8+ T cells.

8. The anti-CNTN4 antibody or antigen-binding fragment thereof of any one of claims 1 to 3, wherein the antigen-binding fragment is selected from the group consisting of Fab, Fab', Fab'-SH, Fv, a single chain antibody scFv, a (Fab')2 fragment, a single domain antibody, a diabody (dAb), and a linear antibody.

9. The anti-CNTN4 antibody or antigen-binding fragment thereof of any one of claims 1 to 3, wherein the antibody is a chimeric antibody or a humanized antibody.

10. The anti-CNTN4 antibody or antigen-binding fragment thereof of any one of claims 1 to 3, wherein the antibody is a multi-specific antibody.

11. The anti-CNTN4 antibody or antigen-binding fragment thereof of any one of claims 1 to 3, wherein the antibody is conjugated with a drug.

12. A nucleic acid molecule encoding the antibody or antigen-binding fragment thereof of any one of claims 1 to 3.

13. A recombinant expression vector comprising the nucleic acid molecule of claim 12.

14. A pharmaceutical composition for preventing or treating cancer, the composition comprising the anti-CNTN4 antibody or antigen-binding fragment thereof of any one of claims 1 to 3.

15. The pharmaceutical composition of claim 14, wherein the cancer is a cancer that expresses CNTN4.

16. A pharmaceutical composition for preventing or treating cancer, the composition comprising the anti-CNTN4 antibody or antigen-binding fragment thereof of any one of claims 1 to 3; and an additional anticancer agent.

17. The pharmaceutical composition of claim 16, wherein the additional anticancer agent is an immune checkpoint inhibitor or a chemotherapeutic agent.

18. The pharmaceutical composition of claim 17, wherein the immune checkpoint inhibitor is one or more selected from the group consisting of anti-CTLA-4 antibodies, anti-PD-1 antibodies, and anti-PD-L1 antibodies.

19. The pharmaceutical composition of claim 16, wherein the anti-CNTN4 antibody or antigen-binding fragment thereof, and the additional anticancer agent are simultaneously administered as a single formulation, or are simultaneously or sequentially administered as a separate formulation.

20. A pharmaceutical composition for preventing or treating cancer, the pharmaceutical composition comprising the anti-CNTN4 antibody or antigen-binding fragment thereof of any one of claims 1 to 3, and being used in combination with an additional anticancer therapy.

21. The pharmaceutical composition of claim 20, wherein the additional anticancer therapy is one or more selected from the group consisting of an immune checkpoint inhibitor, a chemotherapeutic agent, and a radiotherapy.

22. A use of the anti-CNTN4 antibody or antigen-binding fragment thereof of any one of claims 1 to 3 for preventing or treating cancer.

23. A use of the anti-CNTN4 antibody or antigen-binding fragment thereof of any one of claims 1 to 3 for the preparation of a medicament for preventing or treating cancer.

24. A method for preventing or treating cancer, the method comprising administering, to a subject, an effective amount of the anti-CNTN4 antibody or antigen-binding fragment thereof of any one of claims 1 to 3.

25. A method for preventing or treating cancer, the method comprising administering, to a subject, an effective amount of the anti-CNTN4 antibody or antigen-binding fragment thereof of any one of claims 1 to 3, and an effective amount of an additional anticancer agent.

26. The method of claim 25, wherein the additional anticancer agent is an immune checkpoint inhibitor or a chemotherapeutic agent.

27. The method of claim 26, wherein the immune checkpoint inhibitor is one or more selected from the group consisting of anti-CTLA-4 antibodies, anti-PD-1 antibodies, and anti-PD-L1 antibodies.

28. The method of claim 25, an effective amount of the anti-CNTN4 antibody or antigen-binding fragment thereof, and an effective amount of the additional anticancer agent are simultaneously administered to a subject as a single formulation, or are simultaneously or sequentially administered to a subject as a separate formulation.
